# EUROPEAN PATENT SPECIFICATION

(11) **EP 4 380 572 B1**
(45) Date of publication and mention of the grant of the patent: **01.10.2025**
(21) Application number: 22757841.6
(22) Date of filing: 03.08.2022
(51) Int. Cl.: A61K 31/506, A61K 8/87, A61K 9/70, A61P 31/10, A61Q 3/02, A61K 8/34, A61K 8/36, A61K 8/37, A61K 8/49

(54) **FORMULATION FOR SUBSTITUTED 2-AMINOTHIAZOLES IN THE TREATMENT OF FUNGAL AND FUNGAL-BACTERIAL INFECTIONS OF THE NAIL**
FORMULIERUNG FÜR SUBSTITUIERTE 2-AMINOTHIAZOLE BEI DER BEHANDLUNG VON ALLGEMEINEN UND BAKTERIELLEN PILZINFEKTIONEN DES NAGELS
FORMULATION DE 2-AMINOTHIAZOLES SUBSTITUÉS DANS LE TRAITEMENT DES INFECTIONS FONGIQUES ET FONGO-BACTÉRIENNES DE L'ONGLE

(30) Priority: 04.08.2021 EP 21000212
(43) Date of publication of application: 12.06.2024
(73) Proprietor: OnychoPharm GmbH, 13507 Berlin (DE); epinamics GmbH, 14195 Berlin (DE)
(72) Inventor: BREMBECK, Felix, 10785 Berlin (DE); BAASNER, Bernd, 51467 Bergisch Gladbach (DE); FUGMANN, Burkhard, 40878 Ratingen (DE); THIEDE, Hans-Michael, 14773 Potsdam (DE); KEHR, Wolfgang, 14195 Berlin (DE)
(74) Representative: Gruber, Daniel
(86) International application number: PCT/EP2022/000073
(87) International publication number: WO 2023/011750

(56) References cited:
- EP-A1- 3 366 277
- WO-A2-2015/081904
- US-A1- 2007 059 261
- ANONYMOUS: "York Pharma PLC Release: New Abasol(TM) Formulation for Onychomycosis can Effectively and Rapidly Penetrate the Nail Barrier | BioSpace", 31 October 2007 (2007-10-31), pages 1 - 7, XP055877719, Retrieved from the Internet <URL:https://www.biospace.com/article/releases/york-pharma-plc-release-new-abasol-formulation-for-onychomycosis-can-effectively-and-rapidly-penetrate-the-nail-barrier-/> [retrieved on 20220111]
- ANONYMOUS: "ICTRP Search Portal Advanced Search", 1 January 2007 (2007-01-01), pages 1 - 1, XP055877742, Retrieved from the Internet <URL:https://trialsearch.who.int/AdvSearch.aspx> [retrieved on 20220111]
- GUPTA A.K. ET AL: "Onychomycosis: a review", vol. 34, no. 9, 5 June 2020 (2020-06-05), NL, pages 1972 - 1990, XP055874159, ISSN: 0926-9959, Retrieved from the Internet <URL:https://onlinelibrary.wiley.com/doi/full-xml/10.1111/jdv.16394> DOI: 10.1111/jdv.16394

## Description

### FIELD OF THE INVENTION

A formulation for substituted 2-aminothiazoles in the prophylaxis and treatment of fungal and fungal-bacterial infections of the nail (onychomycosis) is disclosed. These formulations are particularly suitable for the topical application onto a nail. After evaporation of the solvent a thin polymer layer is formed on the nail from which the substituted 2-aminothiazole diffuses into the nail and the nail bed. A particularly effective substituted 2-aminothiazole is abafungin. The substituted 2-aminothiazoles are not only fungicidal, but also sporicidal and anti-bacterial. This is a novel approach to avoid frequent relapses in onychomycosis treatment.

### BACKGROUND OF THE INVENTION

Fungal infections of the skin can often be treated effectively by topical administration of antimycotic pharmaceutically active agents. In contrast, there is only modest success in the treatment of fungal and fungal-bacterial infections of the nails. It has proven difficult to deliver antimycotic pharmaceutically active agents effectively into the nails or into the nail bed where the cause of these infections of the nails originates.

Fungal infections in, under and around fingernails and toenails are generally referred to as onychomycosis. Onychomycosis is most frequently caused by dermatophytes such as *Trichophyton rubrum, Trichophyton mentagrophytes* and *Epidermophyton floccosum,* but can also be caused by other types of fungi including molds such as *Fusarium spec* and *Onychocola canadensis,* yeasts, above all *Candida spec* such as *Candida parapsilosis, Candida guilliermondii* and *Candida albicans,* and others. Mixed infections can also occur. Onychomycosis causes thickening, roughness, splitting and discoloration of the nail and can even result in its loss or destruction. In addition, it can be the cause of pain, inadequate blood circulation, problems with walking, and other undesirable symptoms.

One method of treating onychomycosis is to remove surgically the affected part of the nail or even the whole nail. However, this type of treatment can lead to permanent damage to the nail. Also, the newly growing nail can grow in a misshaped form. Moreover, there is no guarantee that the onychomycosis can be completely cured by removing the nail due to recurrence of the infection.

The standard therapy for modest to severe cases of onychomycosis so far is to treat onychomycosis with a variety of antifungal agents, which are administered orally. However, only a small amount of the antifungal agent reaches the nail via the nail matrix. Treatment time is minimum 12 weeks for toenails and about 6 to 8 weeks for fingernails. Oral therapies suffer from many severe adverse side effects, such as irritation of the gastrointestinal tract, nausea, hepatotoxicity, undesirable interactions with other medicaments, skin rashes etc. The oral treatment of onychomycosis is further rendered difficult by individually variable rates of absorption and metabolism. In general, oral long-term treatment with antifungal agents affects liver and kidney function.

Another method of treating onychomycosis for less severe cases is the topical application of a pharmaceutical formulation containing an antifungal active agent, for example, nail lacquers (nail varnishes). However, formulations with nail lacquers cannot efficiently penetrate into the nail to reach the fungal infection, since the nail is a difficult diffusion barrier. Moreover, topical treatment requires at least 48 weeks. This makes the treatment expensive and reduces patient compliance.

US 5,120,530 discloses an antimycotic nail varnish that comprises an antimycotic agent (a morpholine derivative) and a water-insoluble film former, a co-polymer of acrylic acid esters and methacrylic acid esters having a low content of quaternary ammonium groups. This composition is probably poorly effective because it does not contain a keratolytic agent or a humectant. As a result, the nail permeability and consequently the penetration of the antimycotic agent will be very low.

US. 4,957,730 discloses a nail varnish comprising a water-insoluble film-forming substance and an antimycotic agent, specifically 1-hydroxy-2-pyridones. The formulations do not contain a humectant. The nail permeability of these formulations is expected to be very low.

US 5,814,305 discloses a nail preparation comprising an antifungal agent, at least one hydrophilic penetration agent, and a water-alcohol solvent medium. These formulations are in the form of a lotion or fluid gel and do not contain a film-forming agent. Therefore, a sustained release cannot be achieved with these formulations. The need for multiple applications of the formulation might lead to poor patient compliance. The hydrophilic lotions or gels will likely be washed off or removed from the nail in the presence of water or mechanical contact, thereby reducing the accumulation of the active agent in the nail.

GB 2202743 A discloses a topical antifungal composition in the form of a lotion, gel or varnish, comprising at least 1 wt.-% miconazole nitrate or econazole nitrate dissolved in a mixture of water, urea, and a water-soluble dissolving intermediary. Urea is used for increasing the solubility. Such a varnish contains a resin. The disadvantages are the same as for US 5,814,305. Apparently, this delivery system is only suitable for the use of miconazole and econazole.

US 5,346,692 discloses a nail lacquer for treating onychomycosis, which comprises (a) a film-forming agent, (b) at least one antimycotic active agent, (c) urea; and (d) a solvent which comprises (i) 50-70 wt. % of acetone; and (ii) 30-50 wt. % ethanol. This formulation disclosed needs high concentrations of the antimycotic active agent and urea. Adverse effects such as irritation and burning are to be expected.

A major problem in onychomycosis treatment are relapses. Even after an apparently successful treatment in many cases the onychomycosis reappears after some time. This is due to residual fungal spores in the deeper layers of the nail or the nail matrix where they are relatively protected against adverse conditions and oxygen. These resisting, non-dividing spores are not efficiently eliminated by several antifungal agents that lack sporicidal activity. Stimuli such as warmth, humidity and poor oxygen content triggers theses spores to germinate, and onychomycosis reappears. This is the reason why many established antifungal agents show only a poor efficacy in onychomycosis unless they are at the same time sporicidal. The use of an antifungal and sporicidal agent is thus desirable.

A further complication are co-infections, in particular yeasts (*Candida sp*.). Also bacterial infections of the nails occur. They are caused by gram-negative bacteria such as *Pseudomonas aeruginosa* and *Klebsiella spp.* and gram-positive bacteria such as *Staphylococcus aureus.* Moreover, there exist bacterial infections that affect the skin adjacent to the nail. This condition is called paronychia. It is usually caused by an infection with *Staphylococcus aureus,* sometimes also as co-infection with *Candida albicans.* Co-infections of the nail are often opportunistic infections that come after a local weakening of the immune system through a fungal infection. Thus, it is also desirable to use an antifungal agent that treats these co-infections too.

Accordingly, there remains a need for the effective treatment of diseases, disorders and pathological conditions of the nail such as onychomycosis. It would be advantageous to have a topical formulation of an antifungal substance that is capable of penetrating the nail barrier and capable of effectively treating nail fungal diseases, thus avoiding oral administration of anti-fungal agents and the necessity of removing the nail. To be effective, a topical treatment for onychomycosis should exhibit a powerful potency against nail fungi, be at the same time sporicidal and be effective against bacterial co-infections. Moreover, such therapy should also reduce the time of treatment and the relapse of the nail infection.

### DESCRIPTION

Surprisingly, this problem is solved by substituted 2-aminothiazoles dissolved in a polyurethan-containing liquid formulation to be applied on the infected nail.

Disclosed are substituted 2-aminothiazoles of the general formula (I) 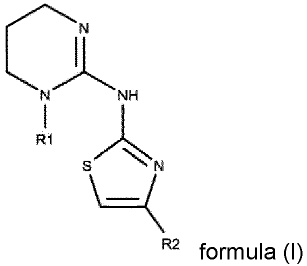
wherein R₁ represents hydrogen or C₁-C₄ straight chain or branched alkyl and R₂ represents a radical of the general formulas (II) 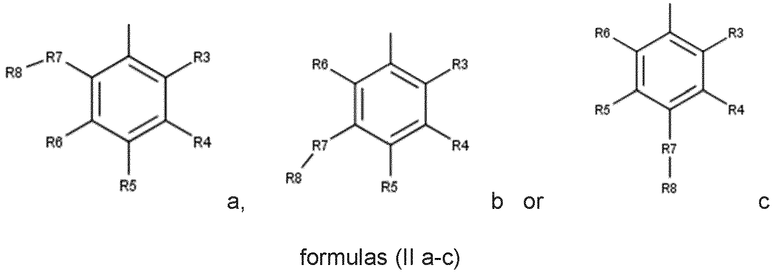
wherein R₃, R₄, R₅ and R₆ independently of one another in each case represent hydrogen, halogen, nitro, alkyl, alkoxy, alkoxycarbonyl, dialkylamino, alkylthio, alkylsulfinyl, alkylsulfonyl each having 1 to 4 carbon atoms in the respective alkyl moieties, or halogenoalkyl, halogenoalkoxy, halogenoalkylthio, halogenoalkylsulfinyl or halogenoalkylsulfonyl each having 1 to 4 carbon atoms and 1 to 9 halogen atoms,
R₇ represents oxygen, sulfur, sulfinyl or sulfonyl and R₈ represents an unsubstituted C₆-C₁₀ aryl or a substituted C₆-C₁₀ aryl radical, substituted by halogen or alkyl, alkoxy, alkoxycarbonyl, dialkylamino, alkylthio, alkylsulphinyl or alkylsulphonyl each having 1 to 8 carbon atoms in the respective alkyl moieties, or halogenoalkyl, halogenoalkoxy, halogenoalkylthio, halogenoalkylsulphinyl or halogenoalkylsulphonyl each having 1 to 4 carbon atoms and 1 to 9 halogen atoms, cycloalkyl having 3 to 7 carbon atoms, phenylalkyl or phenoxyalkyl each having 1 to 4 carbon atoms in the alkyl moiety and also phenyl or phenoxy, a tautomer thereof, or a pharmaceutically acceptable acid addition salt thereof.

These substituted 2-aminothiazoles were first disclosed in DE 38 39 758 A1.

Most preferred is 4-[2-(2,4-dimethylphenoxy)phenyl]-N-(1,4,5,6-tetrahydropyrimidin-2-yl)-1,3-thiazol-2-amine (formula (III)). It is also described as N-[4-[2(2,4-dimethylphenoxy)-phenyl]-2-thiazolyl-1,4,5,6-tetrahydro-2-pyrimidinamine (DTTP). This compound is known in the art as abafungin.

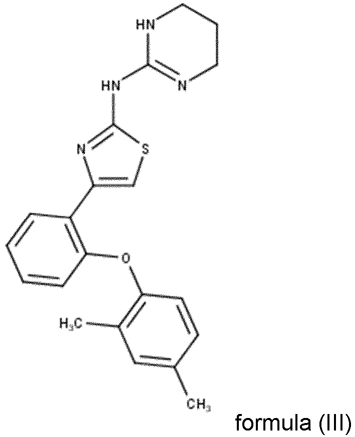

Abafungin displays a crystalline polymorphism. Two anhydrate polymorphic crystalline forms were described in EP 0 718 296 A1. While modification 1 is metastable, modification 2 proved to be photochemically and thermodynamically more stable. It is stable even at high humidity (85% relative humidity) over months. This makes modification 2 the preferred crystalline form and suitable for the use in pharmacological climate zones I to IV. The present disclosure refers to both crystalline forms. However, modification 2 is preferred. Two more crystalline forms of abafungin were disclosed in CN 101845044 A.

The substituted 2-aminothiazoles according to the disclosure can be provided as pharmaceutically acceptable salts of organic and inorganic acids. Suitable examples for these acids are hydrochloric acid, hydrobromic acid, sulfuric acid, phosphoric acid, carbonic acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, digluconic acid, lactic acid, tartaric acid, hydroxymaleic acid, pyruvic acid, phenylacetic acid, benzoic acid, p-aminobenzoic acid, p-hydroxybenzoic acid, dinitrobenzoic acid, chlorbenzoic acid, methanesulfonic acid, ethanesulfonic acid, nitric acid, hydroxyethanesulfonic acid, ethylenesulfonic acid, phenolsulfonic acid, p-toluylsulfonic acid, naphthylsulfonic acid, sulfanilic acid, camphorsulfonic acid, alginic acid, capric acid, hippuric acid, pectinic acid, phthalic acid, quinic acid, pidolic acid, mandelic acid, o-methyl mandelic acid, hydrogen benzenesulfonic acid, picric acid, adipic acid, cyclopentane propionic acid, D-o-toluyl tartaric acid, tartronic acid, benzenesulfonic acid, alpha-methyl benzoic acid, (o, m, p-)methyl benzoic acid, naphthylamine sulfonic acid, phytic acid, tannic acid, boric acid, silicic acid, stannic acid, titanic acid as well as salts from other mineral acids or carbonic acids well known to a person skilled in the art. These salts are generated by contacting the free base with a sufficient amount of the respective acid in order to build the salt in a conventional manner.

Like all of the substituted 2-aminothiazoles according to the disclosure, abafungin is a broadspectrum antifungal agent with a specific mechanism of action. It has been successfully tested for use in the treatment of dermatomycoses such as *Tinea pedis* infections (EudraCT 2005-002842-20).

Abafungin is an achiral compound having a molecular weight of 378.49 g/mol. It shows fungicidal and fungistatic effects on a wide variety of pathogens, including dermatophytes, yeasts (*Candida*) and molds (cf. Borelli et al. (2008) Chemotherapy 54: 245-259). The drug acts on the infecting organisms in two ways: By interfering with the formation of ergosterol in the fungal cell membrane, thereby preventing cell growth, and also by direct interaction with another membrane component resulting in membrane disruption, leakage of cellular contents and death of the cell (sterol 24-C-methyltransferase; Ruiz Ortega et al. (1995) Kidney International 48: 1778-1791; cf. Borelli et al. (2008) Chemotherapy 54: 245-259). This mechanism is independent of whether it is in a non-metabolizing ('resting') phase of development or actively growing. Therefore, abafungin is also sporicidal, in contrast to many other antifungal compounds. Abafungin showed to be bactericidal concentrations in the skin in a 1% preparation. It has a long residence at the site of application (cf. Borelli et al. (2008) Chemotherapy 54: 245-259).

A method for the preparation of abafungin and its analogues was revealed in CN 101372486 A. Recently, a new method for preparing functionalized thiazole heterocyclic compounds through Cu-(I)-catalyzed multi-component cyclization reaction was disclosed. This method of synthesis covers also abafungin (CN 111217767 A).

US 2007/0059261 A1 discloses antimycotic nail polish formulations. These formulations are for use with 2-tetrahydro pyrimidinylamino-thiazole derivatives, particularly abafungin, for the treatment of onychomycoses. Such a formulation was developed by York Pharma under the brand name Abasol^{™} (BioSpace, XP055877719). However, the polyurethane formulations of the disclosure, in particular Liqui-Patch^{®}, are not mentioned.

EP 3366277 A1 discloses film-forming polyurethane formulations comprising organic acids for mycotic infections of the nail. The therapeutic effect is achieved by a lowering of the pH. Substituted 2-aminothiazoles are not mentioned.

WO 2021/5081904 A2 discloses likewise film-forming polyurethane formulations which may include an antimycotic agent for use in humans and animals. Substituted 2-aminothiazoles are not mentioned.

According to the International Clinical Trials Registry Platform of the WHO (XP055877742) no clinical trials with abafungin in the treatment of onychomycoses or with abafungin in a polyurethane matrix have been carried out until now.

In another embodiment of the formulation of the disclosure the formulation further comprises at least one keratolytic agent. A keratolytic agent may help by increasing the penetration of the substituted 2-aminothiazole according to the disclosure into the deeper layers of the nail.

This keratolytic agent can be selected from a group comprising urea, alkali, sulfur, salicylic acid, allantoin, resorcinol, olive oil, acitretin, adapalene, tretinoin, isotretinoin, motretinide, tazarotene, azelaic acid, benzoyl peroxide, podophyllum resin and hexachlorophene.

Additionally, salicylic acid shows bacteriostatic and anti-inflammatory effects. Benzoyl peroxide is known as an antimicrobial agent.

The substituted 2-aminothiazoles according to the disclosure, in particular abafungin, showed an excellent efficacy in models of human onychomycosis when administered in the following formulation according to the disclosure.

This pharmaceutical composition contains:
a) at least one substituted 2-aminothiazole or of its pharmaceutically acceptable salts according to the disclosure;
b) a completely reacted, carboxylated linear polyurethane, comprising the reaction product that consists of
   (1) one or more 2,2-hydroxymethyl-substituted carboxylic acid(s), produced by the general formula (IV), 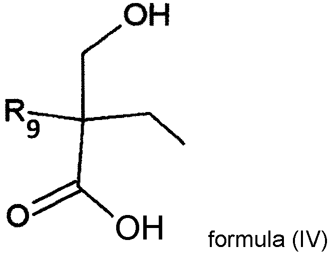 in which R₉ means hydrogen or C₁-C₂₀ alkyl, present in an amount by weight that is sufficient to produce 0.35-2.25 milliequivalents of carboxyl functionality per gram of polyurethane,
   (2) 10-90% by weight, relative to the weight of polyurethane, of one or more organic compound(s), which in each case do not have more than two active hydrogen atoms, and
   (3) one or more organic diisocyanate(s), present in an amount that is sufficient to react with the active hydrogen atoms of the 2,2-hydroxymethyl-substituted carboxylic acid and the organic compounds, with the exception of hydrogen, to form the carboxylate of the 2,2-hydroxymethyl-substituted carboxylic acid;
c) at least one organic or inorganic acid, optionally a methyl, ethyl, propyl or isopropyl ester of said acid(s);
d) an organic solvent;
e) a C₂-C₄ alcohol;
f) H₂O;
g) a plasticizer; and
h) optionally, at least one pharmaceutically acceptable excipient, and/or at least one propellent.

It is preferred that the components a) to h) have in this pharmaceutical composition the following ranges, in respect with the total composition:
a) at least one substituted 2-aminothiazole or of its pharmaceutically acceptable salts in the overall range of 0,5 % to 15% per weight,
b) completely reacted, carboxylated linear polyurethane in the overall range of 0.5% to 4% per weight,
c) at least one organic or inorganic acid in the overall range of 3% to 8% per weight,
d) organic solvent in the overall range of 5% to 25% per weight,
e) C₂-C₄ alcohol in the overall range of 40% to 75% per weight,
f) H₂O in the overall range of 7% to 14% per weight,
g) plasticizer in the overall range of 0.5% to 0.85% per weight, and
h) at least one pharmaceutically acceptable excipient, and/or at least one propellent in the overall range of 0% to 15% per weight,
wherein the relative weight percentages of all ingredients add up to 100 %.

In a more preferred embodiment, the components a) to h) have in this pharmaceutical composition the following ranges, in respect with the total composition:
a) abafungin or one of its pharmaceutically acceptable salts in the overall range of 1% to 10% per weight,
b) completely reacted, carboxylated linear polyurethane in the overall range of 1% to 3% per weight,
c) at least one organic or inorganic acid in the overall range of 4% to 7% per weight,
d) organic solvent in the overall range of 10% to 20% per weight,
e) ethanol in the overall range of 45% to 70% per weight,
f) H₂O in the overall range of 8% to 12% per weight,
g) plasticizer in the overall range of 0.6% to 0.85% per weight, and
h) at least one pharmaceutically acceptable excipient, and/or at least one propellent in the overall range of 0% to 15% per weight,
wherein the relative weight percentages of all ingredients add up to 100 %.

In an even more preferred embodiment, the components a) to h) have in this pharmaceutical composition the following ranges, in respect with the total composition:
a) abafungin or one of its pharmaceutically acceptable salts in the overall range of 2% to 5% per weight,
b) polyurethane-14-AMP-acrylate copolymer in the overall range of 1.3% to 2.7% per weight,
c) at least one organic or inorganic acid in the overall range of 5% to 6.5% per weight,
d) ethyl acetate in the overall range of 10% to 15% per weight,
e) ethanol in the overall range of 50% to 65% per weight,
f) H₂O in the overall range of 8.5% to 11% per weight,
g) plasticizer in the overall range of 0.75% to 0.85% per weight, and
h) at least one pharmaceutically acceptable excipient, and/or at least one propellent in the overall range of 0% to 15% per weight,
wherein the relative weight percentages of all ingredients add up to 100 %.

Surprisingly, it was found that the 2-aminothiazole according to the disclosure, in particular abafungin can be dissolved in the presence of an acid in a solution of the film-forming polyurethane in unexpectedly high amounts. This allows for high concentrations of the antifungal agent in the solution and consequently in the film to be applied on the diseased nail.

A further aspect is that the 2-aminothiazole according to the disclosure, in particular abafungin is particularly well absorbed into the fungal cells at an acid pH. Thus the 2-aminothiazole according to the disclosure, in particular abafungin can exert better fungicidal, respectively fungistatic actions against the microorganisms responsible for onychomycosis in humans. Therefore, the combination of the components according to the disclosure leads to a much higher efficacy in the treatment of onychomycosis in humans.

Specific examples of the carboxylic acid comprise 2,2-di(hydroxymethyl) acetic acid, 2,2-di(hydroxymethyl) propionic acid, 2,2-di(hydroxymethyl) butyric acid, 2,2-di(hydroxymethyl) pentanoic acid and the like. Preferred is 2,2-di(hydroxymethyl) propionic acid. The 2,2-di(hydroxymethyl) substituted carboxylic acids are used in a ratio of 0.35 - 2.25, preferred 0.5 - 1.85 milliequivalents of carboxylic functionality per g polyurethane. In general, this accounts for 5 - 30 % per weight of the polyurethane.

The organic compounds reacting with the isocyanate that are used in the production of the polyurethane polymer according to the disclosure do not have more than two active hydrogen atoms (according to the Zerevitinov determination). These active hydrogen atoms are usually bound to oxygen, nitrogen or sulfur atoms. These compounds have a molecular weight in the range of ca. 300 to 20,000 g/mol, preferred ca. 500 to 8,000 g/mol. Preferably the compounds are linear in order to avoid a gelation during polymerization. But small amounts of non-linear compounds could be used under the condition that their use does not lead to gelation. The organic compounds are present in an amount of 10% - 90% per weight, preferred 15% to 70% per weight of the polyurethane.

The preferred organic compounds with two active hydrogen atoms are the linear bifunctional polyethylene and polypropylene glycols, in particular those that are commercially available and are produced by reaction of ethylene oxide (or propylene oxide) with water, ethylene glycol (or propylene glycol) or diethylene glycol (or dipropylene glycol) in the presence of sodium hydroxide as a catalyst. These polyglycols have molecular weights of about 600 to 20,000 g/mol, preferably about 1,000 to 8,000 g/mol. Polyglycols with homogeneous molecular weight or a mixture of glycols with different molecular weights can be used. It is also possible to incorporate small amounts of additional alkylene oxides by copolymerization into the polyglycol.

Other suitable organic compounds with two active hydrogen atoms are those that have hydroxyl, carboxyl, amino or mercapto groups. Among them, polyhydroxy compounds, such as polyether diols, polyester diols, polyacetal diols, polyamide diols, polyester polyamide diols, poly(alkylene ether) diols, polythioether diols and polycarbonate diols are preferred. Compounds with two or more different groups within these classes, for example amino alcohols and amino alcohols with two amino groups and one hydroxyl group, can also be used. Bifunctional compounds are preferably used, although small amounts of tri- (and multi-) functional compounds can also be used.

Suitable polyether diols are, for example, the condensation products that consist of ethylene oxide, propylene oxide, butylene oxide or tetrahydrofuran and their co-, graft or block polymerization products, such as mixed ethylene oxide, propylene oxide condensates and the graft polymerization products from the reaction of olefins that are under high pressure with the mentioned alkylene oxide condensates. Suitable polyethers are produced by condensation of the above-mentioned alkylene oxides with multivalent alcohols, such as ethylene glycol, 1,2-propylene glycol and 1,4-butanediol.

Suitable polyester diols, polyester amide diols, and polyamide diols are preferably saturated and are obtained by, for example, reaction of saturated or unsaturated polycarboxylic acids with saturated or unsaturated multivalent alcohols, diamines or polyamines. For the production of these compounds, suitable carboxylic acids comprise, for example, adipic acid, succinic acid, phthalic acid, terephthalic acid, and maleic acid. For the production of polyesters, suitable multivalent alcohols comprise, for example, ethylene glycol, 1,2-propylene glycol, 1,4-butanediol, neopentyl glycol and hexanediol. Amino alcohols, such as ethanolamine, are also suitable. For the production of polyester amides and polyamides, suitable diamines are, for example, ethylenediamine and hexamethylenediamine.

Suitable polyacetals can be produced from, for example, 1,4-butanediol or hexanediol and formaldehyde. Suitable polythio ethers can be produced by, for example, condensation of thiodiglycol alone or combined with other glycols, such as ethylene glycol, 1,2-propenylene glycol, or with other polyhydroxy compounds, as previously disclosed. Polyhydroxy compounds, which already contain urea or urethane groups, and natural, multivalent alcohols, which can be further modified, for example castor oil and carbohydrates, can also be used.

In the production of the polyurethane polymer, it may be desirable to subject the polymer, in addition to the organic compound, with not more than two active hydrogen atoms, which in many cases has a high molecular weight, with use of an organic compound with a lower molecular weight, preferably of less than about 300 and more than 60, to a chain lengthening. Typical chain-lengthening agents comprise saturated or unsaturated glycols, such as ethylene glycol, diethylene glycol, triethylene glycol, etc.; amino alcohols, such as ethanolamine, propanolamine, butanolamine, etc.; mono- and di-alkoxylated aliphatic, cycloaliphatic, aromatic and heterocyclic primary amines, such as N-methyl diethanolamine, N-oleyl diethanolamine, N-cyclohexyl diisopropanolamine, N,N-dihydroxyethyl-p-toluidine, N,N-dihydroxypropylnaphthylamine, etc.; diamines, such as ethylenediamine, piperazine, N,N-bis-gamma-aminopropyl-N-methylamine, etc.; carboxylic acids, including aliphatic, cycloaliphatic, aromatic and heterocyclic dicarboxylic acids, such as oxalic acid, malonic acid, succinic acid, glutaric acid, adipic acid, sebacic acid, terephthalic acid, naphthalene-1,5-dicarboyxlic acid, maleic acid, fumaric acid, diglycolic acid, quinolinic acid, lutidinic acid, etc.; aminocarboxylic acids, such as glycine, alpha- and beta-alanine, 6-aminocaproic acid, 4-aminobutyric acid, p-aminobenzoic acid, and 5-aminonaphthoic acid, etc. The preferred chain-lengthening agents are aliphatic diols.

The organic polyisocyanates or mixtures of polyisocyanates, which can be reacted with the organic compound, are aliphatic or aromatic polyisocyanates or mixtures thereof. The polyisocyanates are preferably diisocyanates, thus a linear polymer is produced, although small amounts of trifunctional isocyanates together with the diisocyanates can be used. The isocyanate is present in an amount that is sufficient to react with the active hydrogen atoms of the 2,2-hydroxymethyl-substituted carboxylic acid and the organic compounds, with the exception of hydrogen, to form the carboxylate of 2,2-hydroxymethyl-substituted carboxylic acid. This amount varies, based on the amount of carboxylic acid and organic compounds.

Examples of diisocyanates comprise, without, however, being limited thereto, methylenedi-p-phenyldiisocyanate, methylene-bis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 4,4'-dibenzyl diisocyanate, 1,3-phenylene diisocyanate, 1,4-phenylene diisocyanate, mixtures of 2,4- and 2,6-toluene diisocyanate, 2,2'-dichloro-4,4'-diphenylmethane diisocyanate, 2,4-dibromo-1,5-naphthalene diisocyanate, butane-1,4-diisocyanate, hexane-1,6-diisocyanate, and cyclohexane-1,4-diisocyanate.

If it is not desired to lengthen the polymer chain, the reaction of the diisocyanate with the organic compound, which contains two active hydrogen atoms, is brought to a halt by adding a monofunctional compound that contains active hydrogen in order to consume any residual isocyanate functionality. Examples of these chain stoppers are well known in this area of expertise; for this system, the preferred chain stopper is ethanol.

The polymerization of urethanes is performed in the reaction medium with or without, typically, catalysts for the urethane reaction that are known in the area of expertise. Suitable catalysts comprise dibutyltin-dilaurate, tin (II) salts of carboxylic acids with 2 to 18 carbon atoms, such as tin (II) laurate, tin (II) stearate, tin (II) acetate, tin (II) butyrate, tin (II) octoate, etc., as well as mixtures thereof. The other suitable catalysts include dibutyltin oxide, dibutyltin sulfide, lead resinate, lead benzoate, lead salicylate, lead-2-ethyl hexoate, lead oleate, iron acetyl acetonate, cobalt benzoate, tetra(2-ethylhexyl)-titanate, tetrabutyl titanate, etc. Many other compounds accelerate the reaction of a hydroxyl group or another group with an isocyanate rather than certain other reactions of the isocyanate group, and any of these compounds can be used. The experts are to select a special catalyst to impart desirable characteristic features to the individual urethane reactions. The existing special compounds are the preferred compounds and are mentioned as explanatory and not limiting. In addition, any suitable tertiary amine, for example triethylenediamine, N-ethylmorpholine, N-methylmorpholine or 4-dimethylamino-ethylpiperazine, can be used either alone or together with the metal catalyst.

In regard of the ratio of the co-reactants it should be selected such that the molecular ratio of the isocyanate groups to the active hydrogen atoms is as close as possible to 1:1. Certainly, this exact ratio cannot always be achieved in practice; therefore, a ratio of between about 0.7:1 and 1.3:1 and preferably between about 0.9:1 and 1.2:1 should be sought, and any excess diisocyanate can, as previously discussed, be suppressed with the monofunctional compound that contains active hydrogen.

The polymerization is performed according to known polymerization processes for the production of polyurethane that are well known to the experts in the field. By way of example, polymerization processes and reaction conditions are indicated in the examples of DE 69401230 T2.

Suitable polyurethanes or polyurethane compositions according to the disclosure are
Luviseto P.U.R. (polyurethane-1)
DynamX^{®} (polyurethane-14-AMP-acrylate copolymer),
Avalure^{®} UR 405, Avalure^{®} UR 425 (polyurethane-2),
Avalure^{®} UR 445 (polyurethane-4),
Avalure^{®} UR 450 (polypropyleneglycol-17/isophorone diisocyanate/dimethylpropionic acid copolymer)
Aquamere^{™} A/H (polyvinylpyrrolidone/polycarbamyl/polyglycol ester)
Aquamere^{™} C
(polyvinylpyrrolidone/dimethylaminoethylmethacrylate/polycarbamyl/polyglycol ester)
Aquamere^{™} S (polyvinylpyrrolidone/dimethiconylacrylate/polycarbamyl/polyglycol ester).

Preferred are polyurethane acrylate copolymers. Particularly preferred is polyurethane-14-AMP-acrylate copolymer (contained in DynamX^{®}).

It showed that the substituted 2-aminothiazoles can be particularly pre-solved when exposed to an acidic environment. The respective experiments with abafungin in different acids are presented in Example 2. In Example 2.2 particularly useful combinations were tested.

Component c) of the composition is therefore chosen in regard of nature and quantity so that the resulting pH is in the range of 3.0 to 7.0, preferred 4.0 - 6.0, particularly preferred 4.5 - 5.5, most preferred 4.8 - 5.2.

This component c) can be selected from a group comprising hydrochloric acid, trichloroacetic acid, boric acid, phosphoric acid, formic acid, acetic acid, glyoxylic acid, pyruvic acid, fumaric acid, ascorbic acid, oxalic acid, tannic acid, propionic acid, glycolic acid, lactic acid, lauric acid, or mixtures thereof. Preferred are hydrochloric acid, phosphoric acid, acetic acid, glyoxylic acid, pyruvic acid, oxalic acid, propionic acid, glycolic acid, lactic acid, lauric acid, or mixtures thereof. In principle, any pharmaceutically acceptable acid suitable for a topical application can be used. Additionally, one or more esters of the aforementioned acids can be contained therein. The ester is selected from methyl, ethyl, propyl or isopropyl esters.

Component c) is selected in nature and quantity in such a way that component a) (without propellent) is dissolved in the composition in an amount of more than 10 mg/ml, preferred more than 20 mg/ml, more preferred more than 40 mg/ml, even more preferred more than 60 mg/ml and most preferred more than 100 mg/ml.

The solvent of component d) can be selected from a group consisting of N-methyl pyrrolidone, DMSO, ethyl acetate, butyl acetate and PEG600 (polyethylene glycol 600), and/or mixtures thereof.

As shown in Example 1, these solvents proved to be the most suitable for solving the substituted 2-aminothiazoles according to the disclosure, in particular abafungin, and are therefore preferred.

N-methyl pyrrolidone proved to be the solvent with the highest solving capacity for abafungin. However, N-methyl pyrrolidone has a boiling point from 202°C. This does not facilitate its use in a spray formulation where the solvent should evaporate in a reasonable time in order to leave a fine polymer coating from which the substituted 2-aminothiazole is released onto and into the nail.

DMSO similarly has a high boiling point (189°C). Moreover, many people experience the smell of DMSO as extremely disgusting. This does not contribute to patient compliance. Moreover, there are concerns about the use of DMSO in pharmaceutical compositions. Though this is a topical application where these concerns become less important DMSO would not be the first choice.

PEG600 has a fusion point in the range of 17°C - 22°C. It has a pasty consistency and does not evaporate at room temperature. Hence, it is not the first choice for generating a fine polymer coating from which the substituted 2-aminothiazole is going to be released.

Therefore, ethyl acetate and butyl acetate are more preferred. Ethyl acetate is most preferred, as it has the slightly lower boiling point (77°C vs. 126°C).

Component e) is a C₂-C₄ alcohol. It can be selected from the group comprising ethanol, glycol, 1-propanol, 2-propanol, propane-1,2-diol, propane-1,3-diol, glycerol, 1-butanol, 2-butanol, 1,2-butanediol, 1,3-butanediol,1,4-butanediol, or mixtures thereof. Preferred are ethanol and 2-propanol. Most preferred is ethanol.

Component f) is H₂O. Double distilled water, respectively water for injection are preferred.

Preferred is an aqueous mixture with a water content of less than 50 % per weight (in regard of the solvent content). An organic solvent is classified as volatile when its boiling point is below 85°C, 80°C, 70°C or 60°C.

Component g) is a plasticizer. It can be selected from a group comprising triethyl citrate, tributyl citrate, acetyl triethyl citrate, acetyl tributyl citrate, triacetin, dibutyl phthalate, tributyl sebacate, diethyl phthalate, propylene glycol, polyethylene glycol, glycerol and castor oil, or mixtures thereof. Preferred is triethyl citrate.

Suitable galenic excipient(s) for optional component h) are moisturizers, emulsifiers, stabilizers, solubilizers, permeation enhancers, propellants, colorants, preservatives, antioxidants and aromatic substances.

Suitable moisturizers can be selected from the following group comprising glycerol, sorbitol, propylene glycol, polyethylene glycols, petrolatum, cetyl alcohol, cetearyl alcohol, cocoa butter, isopropyl myristate, isopropyl palmitate, lanolin, liquid paraffin, shea butter, silicone oils, stearic acid, stearyl alcohol, castor oil, and/or polyvinyl pyrrolidone as well as combinations thereof.

Suitable emulsifiers can be selected from the following anionic and non-ionic emulsifiers: Nacetylstearyl sulfate, glycerol fatty acid ester, lecithin, fatty alcohols, cholesterol, sorbitan fatty acid ester, polyoxyethylene(POE)-fatty acid ester, POE-fatty acid glycerides, POE-fatty alcohol ethers, anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Stabilizers are substances that can be added to prevent unwanted changes. Though stabilizers are not real emulsifiers they may also contribute to the stability of emulsions, respectively solubilisates. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Suitable surface-active solubilizing agents (solubilizers) are for example diethylene glycol monoethyl ester, polyethyl propylene glycol co-polymers, cyclodextrins such as α- and β-cyclodextrin, glyceryl monostearates, sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates, polyethylene glycols, aminomethyl propanol.

Suitable permeation enhancers can be selected from a group comprising laurocaprams, sulfoxides, terpenes, terpenoids or essential oils such as eucalyptus, chenopodium and ylang-ylang, oleic acid, oleyl alcohol, lauric acid, propylene glycol, propylene carbonate, N-methyl-pyrrolidone, 2-pyrrolidone, R-(+)-limonene, capric acid, myristic acid, isopropyl myristate, isopropyl myristate mono, dimethyl isosorbide (Arlasolve^{®}), dimethyl sulfoxide (DMSO) and its analogues, dimethyl formamide (DMF), azone (1-dodecylazacycloheptan-2-one), polyoxyethylene-2-oleyl ether, polyoxyethylene-2-stearyl ether, 4-decyloxazolidin-2-one, turpentine oil, pine oil, menthol, combination of oleic acid and propylene glycol, preferably in a ratio of 1:1, combination of R-(+)-limonene and propylene glycol, preferably in a ratio of 1:1, or a combination of isopropyl myristate and propylene glycol.

The propellant of optional component h) is preferably dimethyl ether, a chlorofluorocarbon, a chlorofluorocarbon substitute, carbon dioxide, propane, butane, air and/or nitrogen. A useful composition with a propellant is disclosed in Example 3, the application onto a nail in Example 4.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Preservatives for liquid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of sodium metabisulfite, alpha-tocopherol and ascorbyl palmitate.

Suitable aromatic substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alphacampholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamont, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonen, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonen, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the disclosure all of the aforementioned excipients and classes of excipients can be used without limitation alone or in any conceivable combination thereof, as long as the inventive use is not thwarted, toxic actions may occur, or respective national legislations are infracted.

The pharmaceutical compositions according to the disclosure are particularly suitable for topical use.

These pharmaceutical compositions according to the disclosure are particularly suitable for application, spraying or brushing onto a nail suffering from onychomycosis.

It is particularly preferred if this composition contains for components b), e), f) and g) a composition as disclosed in EP 1962815 B1. Such a pre-formulated composition is available as Liqui-Patch^{®} (epinamics GmbH, Berlin, Germany). It is based on DynamX^{®} (Nouryon, Amsterdam, The Netherlands). It provides excellent features for applying the substituted 2-aminothiazole or its pharmaceutically acceptable salts according to the disclosure onto a nail. The solvent (ethanol) evaporates quickly and allows a polyurethane polymer to build a fine layer on the nail. From this layer the substituted 2-aminothiazole according to the disclosure penetrates the nail and develops its fungicidal, sporicidal and bactericidal actions against the infectant.

Liqui-Patch^{®} has the following composition (Table 1):

**Table 1:**

| | |
|---|---|
| polyurethane-14-AMP-acrylate copolymer | 2.8 % by weight |
| H₂O | 14.4 % by weight |
| ethanol | 81.5 % by weight |
| triethyl acetate | 1.0 % by weight |
| aminomethyl propanol | 0.3 % by weight |

The pharmaceutic composition according to the disclosure can be provided as a solution, emulsion, suspension, sprayable composition, in particular for transdermal applications or for applications on the nail plate. A pharmaceutical composition according to the disclosure serves for the prophylaxis or treatment of onychomycosis.

The application refers also to a coating or a layer containing or consisting of a composition according to the disclosure when a volatile solvent has evaporated. Such a coating or layer can be generated for example from a composition according to the disclosure by spreading or spraying it onto a nail while the solvent contained in the composition evaporates.

A pharmaceutical composition according to the disclosure can be produced by mixing components a) and c), optionally also d) first. In a particularly simple embodiment, a first composition containing components a) and c) is mixed with components b) and d). In regard with a composition containing components b), e), f) and g) it is referred to EP 1962815 B1 which herewith is incorporated entirely into the present disclosure.

In another aspect the application refers also to an applicator containing a pharmaceutical composition according to the disclosure, particularly in form of a roller, pump sprayer, sprayer, spray bottle, tube, bottle brush or bottle pipette.

In yet another aspect the application discloses also a method for preparing a pharmaceutical composition according to the disclosure, comprising the following steps:
a) at least one substituted 2-aminothiazole as defined in claim 1 and at least one organic or inorganic acid as defined in claim 5 are mixed under stirring to afford a first solution;
b) the first solution of step a) is mixed under stirring with an organic solvent as defined in claim 6 to afford a second solution;
c) a completely reacted, carboxylated linear polyurethane as defined in claim 1, a C₂-C₄ alcohol as defined in claim 7, H₂O and a plasticizer as defined in claim 8 are mixed under stirring to afford a third solution;
d) the second solution of step b) and the third solution of step c) are mixed under stirring to afford a fourth solution; and
e) optionally, at least one pharmaceutically acceptable excipient as defined in claim 9 or 10 is added to the fourth solution of step d).

The efficacy of pharmaceutical compositions according to the disclosure were tested in two different models of onychomycosis-like infection with *T. rubrum.*

In Example 5, the zone-of-inhibition was determined that an application of a test solution according to the disclosure with abafungin on a human nail clip could effect after 7 days on the fungi *Trichophyton rubrum* cultivated in a petri dish in a modified Franz chamber under the nail. It showed that abafungin had nearly as good results as the eficonazole formulation Jublia^{™}, at the moment the standard drug on the market in North America.

In Example 6, the percentage of surviving fungi (*T. rubrum*) was assessed on an infected human nail clip mounted in a modified Franz chamber. The amount of ATP after a treatment with a test solution according to the disclosure was determined, as an equivalent measure of the surviving fungi. Abafungin showed an excellent dose-dependent efficacy. In this paradigm the efficacy of abafungin was even slightly better than of Jublia^{™}.

Taken together, the formulations according to the disclosure for the substituted 2-aminothiazoles are suitable for the treatment of onychomycosis and the prophylaxis of relapses. The overall efficacy on the primary infection with *T. rubrum* is comparable to the standard medication Jublia^{™}.

Abafungin, as well as the other disclosed substituted 2-aminothiazoles, however, have the advantage that they show in addition sporicidal and anti-bacterial actions. This is not the case for eficonazole and many other antifungal drugs that are usually applied systemically. Therefore, the risk for relapses is relatively high and the treatment has to be repeated after several months. It can be reasonably assumed that this is not the case with the substituted 2-aminothiazoles of the disclosure, in particular abafungin. The fungal spores that usually survive an antifungal treatment have the potency to regrow and thus lead to re-infection of the nail This is heavily suppressed by sporicidal drugs such as the disclosed substituted 2-aminothiazoles, in particular abafungin.

Also the risk of co-infections of the nail with bacteria and/or yeasts will be considerably diminished. While up to now the treatment of these co-infections is hampered in that one would have to use additionally systemically applied drugs to eradicate the co-infections, with the difficulty to reach the nail bed and the nail in effective amounts. In contrast, a relatively short topical treatment with the disclosed substituted 2-aminothiazoles, in particular abafungin promises a solution because of their additional effects. In this light the treatment of onychomycoses and the prophylaxis of relapses with the disclosed substituted 2-aminothiazoles, in particular abafungin have also a positive effect beyond the mere treatment of the acute fungal infection.

### EXAMPLES

Abafungin was provided by OnychoPharm GmbH, Berlin, Germany. DynamX^{®} was purchased from Nouryon, Amsterdam, The Netherlands. Standard chemicals were purchased from Sigma-Aldrich, Germany.

### Example 1: Solubility of abafungin in different solvents

**2 ml of solvent** were placed in a glass vial containing abafungin powder. The vial was mechanically shaken for 24 h in an environment with controlled temperature (19°C). Thereafter the content of the vials was transferred into Eppendorf tubes and centrifuged at 13000 rpm for 3 min. The supernatant (saturated solution) was collected and assayed using a UV spectrometer at 302 nm (λₘₐₓ of abafungin). If the saturated solution had an absorbance > 1, it was diluted with the same solvent to achieve an absorbance < 1, and preferably within the same absorbance range of the absorbance-concentration standard curve with that solvent. Drug concentration in the saturated solution (or diluted solution) was then determined using a standard curve (Table 2). Each experiment was conducted in triplicates. N-methyl pyrrolidone, DMSO, ethyl acetate, butyl acetate and PEG600 proved to be the best solvents for abafungin.

**Table 2:**

| **Solvent** | **Solubility mg/100 ml mean ± standard deviation** |
|---|---|
| water | < 0.032 |
| ethanol | 238.46 ± 2.72 |
| 1,2-propanediol | 219.93 ± 12.10 |
| N-methyl pyrrolidone | 27678.92 ± 434.07 |
| isopropanol | 156.14 ± 16.5 |
| isopropanol myristate | 50.60 ± 4.30 |
| DMSO | 4483.42 ± 16.30 |
| ethyl acetate | 803.75 ± 35.51 |
| butyl acetate | 822.13 ± 2.55 |
| PEG600 | 5029.70 ± 12.70 |

### Example 2: Solubility of abafungin in Liqui-Patch^{®}

### 2.1 Comparative compositions: Solubility of abafungin in different solvents

2.1.1 Abafungin was dissolved overnight in an ethanolic solution of PUAC (polyurethaneacrylate, PUAC/EtOH 1:3 by weight percentages) at 2000 rpm and 25°C. This yielded a concentration of abafungin of 4.26 mg/ml.

2.1.2 Abafungin was dissolved overnight in Liqui-Patch^{®} from the company epinamics GmbH at 2000 rpm and 25°C. This yielded a concentration of abafungin of 1.81 mg/ml.

2.1.3 Abafungin was dissolved for 1 h in Liqui-Patch^{®} from the company epinamics GmbH at 2000 rpm and 25°C. In two experiments, this yielded a concentration of abafungin of 1.88 mg/ml and 0.95 mg/ml, respectively.

2.1.4 Abafungin was dissolved overnight in a mixture of Liqui-Patch^{®} from the company epinamics GmbH and ethyl acetate (5%, percentage by weight) at 2000 rpm and 25°C. This yielded a concentration of abafungin of 2.3 mg/ml.

2.1.5 Abafungin was dissolved overnight in a mixture of Liqui-Patch^{®} from the company epinamics GmbH and ethyl acetate (10%, percentage by weight) at 2000 rpm and 25°C. This yielded a concentration of abafungin of 2.72 mg/ml.

2.1.6 Abafungin was dissolved overnight in a mixture of Liqui-Patch^{®} from the company epinamics GmbH and ethyl acetate (20%, percentage by weight) at 2000 rpm and 25°C. This yielded a concentration of abafungin of 4.09 mg/ml.

2.1.7 Abafungin was dissolved overnight in a mixture of Liqui-Patch^{®} from the company epinamics GmbH and ethyl acetate (40%, percentage by weight) at 2000 rpm and 25°C. This yielded a concentration of abafungin of 7.13 mg/ml.

2.1.8 Abafungin was dissolved for 1 h in a mixture of Liqui-Patch^{®} from the company epinamics GmbH and DMSO (5%, percentage by weight) at 2000 rpm and 25°C. This yielded a concentration of abafungin of 2.02 mg/ml.

2.1.9 Abafungin was dissolved for 1 h in a mixture of Liqui-Patch^{®} from the company epinamics GmbH and ethyl acetate (10%, percentage by weight) at 2000 rpm and 25°C. This yielded a concentration of abafungin of 2.21 mg/ml.

2.1.10 Abafungin was dissolved for 1 h in a mixture of Liqui-Patch^{®} from the company epinamics GmbH and ethyl acetate (20%, percentage by weight) at 2000 rpm and 25°C. This yielded a concentration of abafungin of 2.81 mg/ml.

2.1.11 Abafungin was dissolved for 1 h in water at 2000 rpm and 25°C. This yielded a concentration of abafungin of 0 mg/ml.

2.1.12 Abafungin was dissolved for 1 h in water with 2% fumaric acid at 2000 rpm and 25°C. This yielded a concentration of abafungin of 0 mg/ml.

2.1.13 Abafungin was dissolved for 1 h in water with 10% fumaric acid at 2000 rpm and 25°C. This yielded a concentration of abafungin of 0 mg/ml.

2.1.14 Abafungin was dissolved for 1 h in water with 2% ascorbic acid at 2000 rpm and 25°C. This yielded a concentration of abafungin of 0 mg/ml.

2.1.15 Abafungin was dissolved for 1 h in water with 10% ascorbic acid at 2000 rpm and 25°C. This yielded a concentration of abafungin of 6.4 mg/ml.

2.1.16 Abafungin was dissolved for 1 h in water with 1% boric acid at 2000 rpm and 25°C. This yielded a concentration of abafungin of 0 mg/ml.

2.1.17 Abafungin was dissolved for 1 h in water with 4% boric acid at 2000 rpm and 25°C. This yielded a concentration of abafungin of 0 mg/ml.

### 2.2 Compositions according to the disclosure

It showed that abafungin is dissolved best in Liqui-Patch^{®} formulations when ethyl acetate and an acid are added.

2.2.1 Abafungin was dissolved overnight in a mixture of Liqui-Patch^{®} from the company epinamics GmbH and glacial acetic acid (5%, percentage by weight) at 2000 rpm and 25°C. This yielded a concentration of abafungin of 121.54 mg/ml.

2.2.2 Abafungin was dissolved overnight in a mixture of Liqui-Patch^{®} from the company epinamics GmbH and glacial acetic acid (7.5%, percentage by weight) at 2000 rpm and 25°C. This yielded a concentration of abafungin of > 179.5 mg/ml.

2.2.3 Abafungin was dissolved overnight in a mixture of Liqui-Patch^{®} from the company epinamics GmbH and glacial acetic acid (10%, percentage by weight) at 2000 rpm and 25°C. This yielded a concentration of abafungin of > 136.13 mg/ml.

2.2.4 Abafungin was dissolved for 1 h in a mixture of Liqui-Patch^{®} from the company epinamics GmbH and fumaric acid (0.5%, percentage by weight) at 2000 rpm and 25°C. This yielded a concentration of abafungin of 60.95 mg/ml.

2.2.5 Abafungin was dissolved for 1 h in a mixture of Liqui-Patch^{®} from the company epinamics GmbH and fumaric acid (2%, percentage by weight) at 2000 rpm and 25°C. This yielded a concentration of abafungin of 243.34 mg/ml.

2.2.6 Abafungin was dissolved for 1 h in a mixture of Liqui-Patch^{®} from the company epinamics GmbH and fumaric acid (5%, percentage by weight) at 2000 rpm and 25°C. This yielded a concentration of abafungin of > 200 mg/ml.

2.2.7 Abafungin was dissolved for 1 h in a mixture of Liqui-Patch^{®} from the company epinamics GmbH and fumaric acid (10%, percentage by weight) at 2000 rpm and 25°C. This yielded a concentration of abafungin of > 200 mg/ml.

2.2.8 Abafungin was dissolved for 1 h in a mixture of Liqui-Patch^{®} from the company epinamics GmbH and glyoxylic acid (1%, percentage by weight) at 2000 rpm and 25°C. This yielded a concentration of abafungin of 39.94 mg/ml.

2.2.9 Abafungin was dissolved for 1 h in a mixture of Liqui-Patch^{®} from the company epinamics GmbH and glyoxylic acid (5%, percentage by weight) at 2000 rpm and 25°C. This yielded a concentration of abafungin of 184.39 mg/ml.

2.2.10 Abafungin was dissolved for 1 h in a mixture of Liqui-Patch^{®} from the company epinamics GmbH and pyruvic acid (1%, percentage by weight) at 2000 rpm and 25°C. This yielded a concentration of abafungin of 15.41 mg/ml.

2.2.11 Abafungin was dissolved for 1 h in a mixture of Liqui-Patch^{®} from the company epinamics GmbH and pyruvic acid (5%, percentage by weight) at 2000 rpm and 25°C. This yielded a concentration of abafungin of 91.93 mg/ml.

2.2.12 Abafungin was dissolved for 1 h in a mixture of Liqui-Patch^{®} from the company epinamics GmbH and phosphoric acid (1%, percentage by weight) at 2000 rpm and 25°C. This yielded a concentration of abafungin of 33.77 mg/ml.

2.2.13 Abafungin was dissolved for 1 h in a mixture of Liqui-Patch^{®} from the company epinamics GmbH and phosphoric acid (5%, percentage by weight) at 2000 rpm and 25°C. This yielded a concentration of abafungin of 116.25 mg/ml.

2.2.14 Abafungin was dissolved for 1 h in a mixture of Liqui-Patch^{®} from the company epinamics GmbH and oxalic acid (2%, percentage by weight) at 2000 rpm and 25°C. This yielded a concentration of abafungin of 140.85 mg/ml.

2.2.15 Abafungin was dissolved for 1 h in a mixture of Liqui-Patch^{®} from the company epinamics GmbH and oxalic acid (10%, percentage by weight) at 2000 rpm and 25°C. This yielded a concentration of abafungin of 184.91 mg/ml.

2.2.16 Abafungin was dissolved for 1 h in a mixture of Liqui-Patch^{®} from the company epinamics GmbH and tannic acid (2%, percentage by weight) at 2000 rpm and 25°C. This yielded a concentration of abafungin of 5.88 mg/ml.

2.2.17 Abafungin was dissolved for 1 h in a mixture of Liqui-Patch^{®} from the company epinamics GmbH and tannic acid (10%, percentage by weight) at 2000 rpm and 25°C. This yielded a concentration of abafungin of 36.23 mg/ml.

2.2.18 Abafungin was dissolved for 1 h in a mixture of Liqui-Patch^{®} from the company epinamics GmbH and propionic acid (2%, percentage by weight) at 2000 rpm and 25°C. This yielded a concentration of abafungin of 23.34 mg/ml.

2.2.19 Abafungin was dissolved for 1 h in a mixture of Liqui-Patch^{®} from the company epinamics GmbH and propionic acid (10%, percentage by weight) at 2000 rpm and 25°C. This yielded a concentration of abafungin of 144.76 mg/ml.

2.2.20 Abafungin was dissolved for 1 h in a mixture of Liqui-Patch^{®} from the company epinamics GmbH and glycolic acid (2%, percentage by weight) at 2000 rpm and 25°C. This yielded a concentration of abafungin of 70.4 mg/ml.

2.2.21 Abafungin was dissolved for 1 h in a mixture of Liqui-Patch^{®} from the company epinamics GmbH and glycolic acid (10%, percentage by weight) at 2000 rpm and 25°C. This yielded a concentration of abafungin of 25.52 mg/ml.

2.2.22 Abafungin was dissolved for 1 h in a mixture of Liqui-Patch^{®} from the company epinamics GmbH and lactic acid (2%, percentage by weight) at 2000 rpm and 25°C. This yielded a concentration of abafungin of 44.33 mg/ml.

2.2.23 Abafungin was dissolved for 1 h in a mixture of Liqui-Patch^{®} from the company epinamics GmbH and lactic acid (10%, percentage by weight) at 2000 rpm and 25°C. This yielded a concentration of abafungin of 229.62 mg/ml.

2.2.24 Abafungin was dissolved for 1 h in a mixture of Liqui-Patch^{®} from the company epinamics GmbH and hydrochloric acid (0.2%, percentage by weight) at 2000 rpm and 25°C. This yielded a concentration of abafungin of 13.81 mg/ml.

2.2.25 Abafungin was dissolved for 1 h in a mixture of Liqui-Patch^{®} from the company epinamics GmbH and hydrochloric acid (1%, percentage by weight) at 2000 rpm and 25°C. This yielded a concentration of abafungin of 48.5 mg/ml.

2.2.26 Abafungin was dissolved for 1 h in a mixture of Liqui-Patch^{®} from the company epinamics GmbH and trichloroacetic acid (1%, percentage by weight) at 2000 rpm and 25°C. This yielded a concentration of abafungin of 12.49 mg/ml.

2.2.27 Abafungin was dissolved for 1 h in a mixture of Liqui-Patch^{®} from the company epinamics GmbH and trichloroacetic acid (5%, percentage by weight) at 2000 rpm and 25°C. This yielded a concentration of abafungin of 6.93 mg/ml.

2.2.28 Abafungin was dissolved for 1 h in a mixture of Liqui-Patch^{®} from the company epinamics GmbH and lauric acid (2%, percentage by weight) at 2000 rpm and 25°C. This yielded a concentration of abafungin of 9.51 mg/ml.

2.2.29 Abafungin was dissolved for 1 h in a mixture of Liqui-Patch^{®} from the company epinamics GmbH and lauric acid (10%, percentage by weight) at 2000 rpm and 25°C. This yielded a concentration of abafungin of 54.93 mg/ml.

2.2.30 Abafungin was dissolved for 1 h in a mixture of Liqui-Patch^{®} from the company epinamics GmbH and glyoxylic acid (2%, percentage by weight) at 2000 rpm and 25°C. This yielded a concentration of abafungin of 81.22 mg/ml.

2.2.31 Abafungin was dissolved for 1 h in a mixture of Liqui-Patch^{®} from the company epinamics GmbH, glyoxylic acid (2%, percentage by weight) and ethyl acetate (10%, percentage by weight) at 2000 rpm and 25°C. This yielded a concentration of abafungin of 87.9 mg/ml.

2.2.32 Abafungin was dissolved for 1 h in a mixture of Liqui-Patch^{®} from the company epinamics GmbH, glyoxylic acid (2%, percentage by weight) and ethyl acetate (25%, percentage by weight) at 2000 rpm and 25°C. This yielded a concentration of abafungin of 90.88 mg/ml.

2.2.33 Abafungin was dissolved for 1 h in a mixture of Liqui-Patch^{®} from the company epinamics GmbH and acetic acid (2%, percentage by weight) at 2000 rpm and 25°C. This yielded a concentration of abafungin of 41.61 mg/ml.

2.2.34 Abafungin was dissolved for 1 h in a mixture of Liqui-Patch^{®} from the company epinamics GmbH, acetic acid (2%, percentage by weight) and ethyl acetate (10%, percentage by weight) at 2000 rpm and 25°C. This yielded a concentration of abafungin of 48.22 mg/ml.

2.2.35 Abafungin was dissolved for 1 h in a mixture of Liqui-Patch^{®} from the company epinamics GmbH, acetic acid (2%, percentage by weight) and ethyl acetate (25%, percentage by weight) at 2000 rpm and 25°C. This yielded a concentration of abafungin of 55.79 mg/ml.

### Example 3: Abafungin composition with a propellant

Abafungin is dissolved in 1 part by weight lactic acid in an amount of 1000 mg/ml. This solution is mixed with 9 parts by weight of Liqui-Patch^{®} from the company epinamics GmbH. This yields a composition containing 100 mg/ml abafungin. Optionally, a propellant is added to the resulting mixture. Then the mixture is filled into a spray bottle. The compositions from Example 2.2. can be produced in an analogous manner.

### Example 4: Application of an abafungin composition with a propellant

The composition of Example 3 is brushed or sprayed onto a nail and the volatile solvent contained therein evaporates.

### Example 5: Modified zone-of-inhibition assay (ThurChub^{®})

The nail clipping from healthy human volunteers fulfilling standard criteria (clean, un-diseased, not painted using nail varnish in the last 6 months) were stored in individual containers in a freezer. Prior to use, the nails were removed from the freezer (leaving them in the individual sealed containers) and placed in a biological safety cabinet for 30 min to defrost.

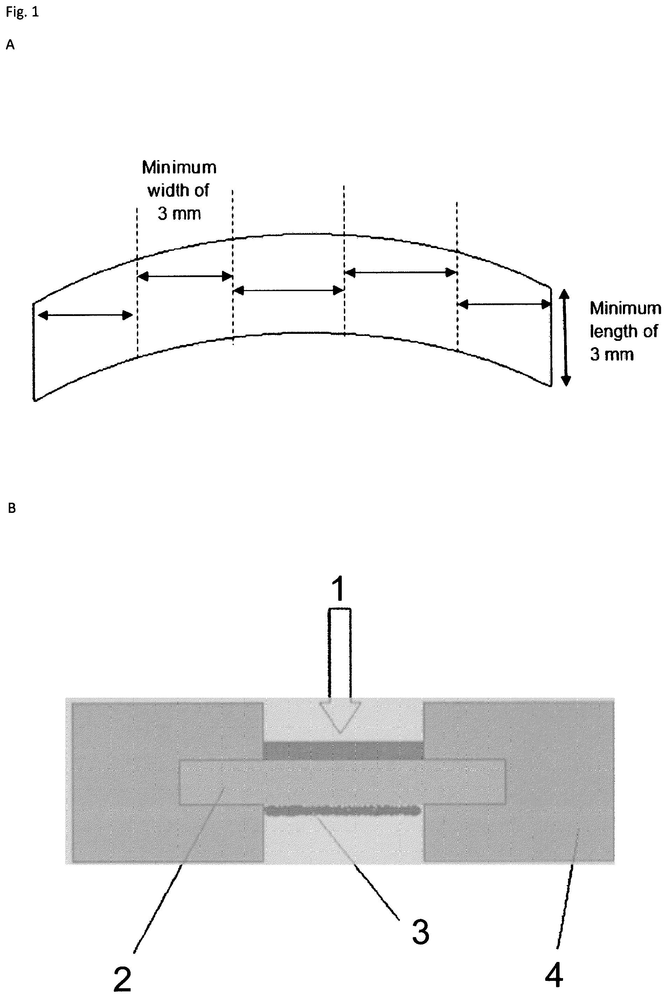

Following defrosting the nails were briefly washed separately as follows:
(i) The nails were initially placed into ambient laboratory temperature deionized water (18.2 MΩ (Milli-Q) or HPLC grade) for 15 min to soften prior to cutting
(ii) The nails were cut into ca. 3 mm x 3 mm sections using scissors (ca. 9 mm² surface area; Fig. 1A) and the thickness of the nail sections were subsequently measured using a pair of calipers and recorded in the study specific laboratory workbook (the scissors and calipers were wiped completely with a 70% v/v ethanol in water solution and left to dry under a biological safety cabinet for 30 min prior to use)
(iii) The nails were immersed into 10 mL of 70% v/v ethanol in water solution and vortex mixed for 1 min
(iv) The 70% v/v ethanol-in-water solution of Step (iii) was decanted, replaced with fresh 70% v/v ethanol-in-water solution and vortex-mixed for a further 1 min
(v) The 70% v/v ethanol-in-water solution of Step (iv) was decanted, replaced with sterile Ringer's solution and vortex-mixed for 1 min
(vi) The Ringer's solution of Step (v) was decanted, replaced with fresh sterile Ringer's solution and vortex-mixed for a further 1 min
(vii) Once the washing process was complete, the nails were transferred into a sterile Petri dish without a lid and air dried under a biological safety cabinet for ca. 30 min at ambient laboratory temperature
(viii) Each nail section was placed carefully into a single well of a sterile 24-well plate using forceps (which were wiped completely with the 70% v/v ethanol-in-water solution and left to dry under a biological safety cabinet for ca. 30 min prior to use)
(ix) The lid of the 24-well plate was placed on top of the plate and the plate was stored at 2 - 8°C until the nails were required

A zone of inhibition (ZOI) assay in TurChub^{®} cells (without a nail barrier; MedPharm, Guildford, UK) and Petri dishes (90 mm) containing each individual media (SDA (Sabouraud dextrose agar medium), PDA (potato dextrose agar medium) and RPMI (glucose-rich Roswell Park Memorial Institute medium) was performed against the selected test organism (*T. rubrum*) using a test formulation of 5% by weight abafungin (Table 3):

**Table 3:**

| | |
|---|---|
| abafungin | 5 % by weight |
| glacial acetic acid | 6 % by weight |
| ethyl acetate | 10 % by weight |
| Liqui-Patch^{®} | 79 % by weight |

This test formulation was applied to a sterile antibiotic disc. This was performed to confirm activity of the test formulations against the test organism, compatibility of the test formulations with the media and to confirm zones of inhibition are obtainable with the formulations prior to investigating efficacy in the TurChub^{®} cells with a nail barrier.

The experiment was performed as follows:
TurChub^{®} cells mounted with full thickness nails were set up using PDA as growth medium for *T. rubrum* and were occluded and incubated at 20°C - 25°C for 7 days following treatment. The zone of inhibition investigation was performed in order to optimize the experimental parameters for the full-scale investigation as follows:
(i) *T. rubrum* suspension was pipetted (50 µl) onto the surface of PDA within individual TurChub^{®} cells and then left to dry under a biological safety cabinet
(ii) Aliquots of the test formulations were applied (100 µl daily for 7 days to the surface of individual nails in TurChub^{®} cells (n=6) using a calibrated automatic pipette and then allowed to dry for 10 min under the biological safety cabinet
(iii) All of the TurChub^{®} test systems were covered and incubated at 20°C - 25°C for 7 days. For daily dosing of the formulation, subsequent additions of test formulation were performed as detailed in Step (ii), however the nails were cleaned with Ringer's solution prior to application. Infected and non-infected controls (n=2) were also included where the infected was inoculated with organism but not treated with any test formulation, and the non-infected control was neither inoculated nor treated with any test formulation.
(iv) The efficacy of the test formulations was determined after 7 days by measuring the zone of inhibition of growth of *T. rubrum* in the TurChub^{®} cell. The zone diameter (if any observed) was measured using calibrated calipers and the data recorded.

The triazole antifungal eficonazole ( ((2R,3R)-2-(2,4-difluorophenyl)-3-(4-methylenepiperidin-1 yl)-1-(1H-1,2,4-triazol-1-yl) butan-2-ol ) was chosen as positive control. Recently, an eficonazole topical solution received the marketing authorization in the USA, Japan and Canada for the treatment of onychomycoses. It is branded under the name Jublia^{™}. Eficonazole acts as a 14-alpha demethylase inhibitor (Tatsumi et al. (2013) Antimicrob Agents Chemother 57: 2405-2409).

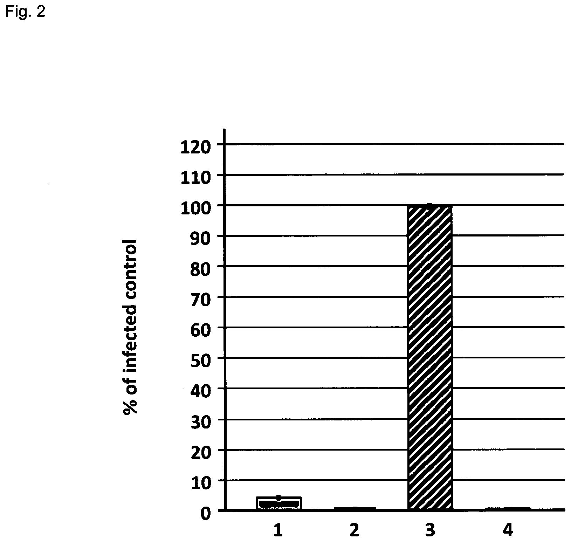

The application of a single dose (100 µl) on Day 1 yielded the following results (see also Fig. 2):

**Table 4:**

| | | | |
|---|---|---|---|
| 1. | 5% abafungin in Liqui-Patch^{®} | 3.45 cm | 4.2 % |
| 2. | 10% eficonazole (Jublia^{™}) | 3.58 cm | 0.6 % |
| 3. | infected untreated control | 0 cm | 100 % |
| 4. | not infected | 3,60 cm | 0% |

In Table 4 the zone-of-inhibition on the infected petri dishes is indicated in cm. The corresponding percentages were normalized. The maximally achievable distance (not infected) was set to 0%, while the infected untreated control was set to 100%. It showed that in this experimental setting abafungin performed nearly as good as eficonazole.

### Example 6: Infected nail model (RoMar^{®})

An ATP cell viability test (RoMar^{™}; MedPharm, Guildford, UK) was carried out in order to determine the percentage of surviving fungi after treatment.

First, abafungin was tested in the ChubTur^{®} test system. In short, a Sabouraud dextrose agar (SDA) slope inoculated with *Trichophyton rubrum (T. rubrum)* was originally isolated from a patient suffering from onychomycosis. The organism was subcultured onto fresh SDA slopes and reference samples were placed into a glycerol solution and cryogenically frozen. The organism has been subcultured on a three monthly basis to maintain viability. The cultures were stored at 20 - 25 °C for seven days after subculturing, and then stored in the fridge at 2 - 8 °C until required. Suspensions of *T. rubrum* were prepared under a biological safety cabinet.

Nail clippings from healthy human volunteers fulfilling standard (clean, un-diseased, not painted using nail varnish in the last 6 months; Fig. 1A) were stored in individual containers in a freezer. Prior to use, the nails were removed from the freezer (leaving them in the individual sealed containers) and placed in a biological safety cabinet for 30 min to defrost. Following defrosting, the nails were briefly washed separately as follows:
(i) The nails were placed into deionized water (18.2 MΩ (Milli-Q) or HPLC grade) and heated to 60 °C for 15 min.
(ii) The nails were cut into ca. 3 mm x 3 mm sections using scissors (ca. 9 mm² surface area) and the thickness of the nail sections were subsequently measured using a pair of calipers and left to dry under a biological safety cabinet for 30 min prior to use.
(iii) The nails were immersed into 10 ml of 70% v/v ethanol-in-water solution and vortex mixed for 1 min.
(iv) The 70% v/v ethanol-in-water solution was decanted, replaced with fresh 70% v/v ethanol-in-water solution and vortex mixed for a further 1 min.
(v) The 70% v/v ethanol in water solution was decanted, replaced with sterile Ringer's solution and vortex mixed for 1 min.
(vi) The Ringer's solution was decanted, replaced with fresh sterile Ringer's solution and vortex mixed for a further 1 min.
(vii) Once the washing process was complete, the nails were transferred into a sterile Petri dish without a lid and air-dried under a biological safety cabinet for ca. 30 min at ambient laboratory temperature.
(viii) Each nail section was placed carefully into a single well of a sterile 24- or 96-well plate using forceps (which were wiped completely with the 70% v/v ethanol-in-water solution and left to dry under a biological safety cabinet for ca. 30 min prior to use).
(ix) The lid of the 24- or 96-well plate was placed on top of the plate and the plate was stored at 2 - 8 °C until the nails were required.

### ATP calibration assay

Adenosine triphosphate (ATP) calibration standards of known concentrations were prepared by sequentially diluting a 1 mg/mL stock ATP standard in Ringer's solution. The total amount of light emitted from each well was then measured and a calibration curve generated. The purpose of this investigation was to identify and demonstrate a trend between the amount of luminescence measured to the amount of ATP present, such that the efficacy of the test solvent systems could subsequently be determined during the *in vitro* infected nail investigation (by comparing luminescence units (LU) of the test formulations to the appropriate controls).

### Quenching effect investigation

The quenching effect of the test formulations on the ATP assay was investigated to ensure the ATP assay was fit for purpose as follows:
(i) The test formulations (2 µl) were transferred to individual wells (n=3) in a 96-well microtiter plate
(ii) A single ATP calibration standard e.g., 55.0 ng/ml (50 µl) was selected (based on potential ATP recovery) and transferred to the wells. Lysing solution (100 µl) was transferred to each well through the Flx800 plate reader, and the plate was read immediately
(iii) The amount of LU emitted from the wells was then compared to a positive control well (50 µl of the ATP calibration standard used only)
(iv) The amount of LU emitted from a negative control (n=1; Ringer's, 50 µl) was also determined
(v) The quenching effect was determined by comparing the percentage recovery of the ATP standard with formulation to the positive control in which no formulation was added

### Infected nail assay

An *in vitro* infected nail investigation against *T. rubrum* was performed using a LiquiPatch^{®} formulation (see above) containing 10% abafungin for optimizing the experimental parameters for the full-scale infected nail investigation. Each section of full thickness nail was infected (where applicable) and incubated in the ChubTur^{®} cell test systems (see above) as follows:
i) Prepared nails were individually infected on the underside with *T. rubrum* using 5 µl of the organism suspension. The suspension was allowed to dry on the nail for 30 min under a biological safety cabinet.
ii) Once the nail was dry, it was mounted onto the nail gasket within the ChubTur^{®} cell. An inert humidity control was added to the receiver chamber of the cell so that an air gap was maintained through the sampling arm (sterile Ringer's solution). A humidity control was also added to the surface of the nail mounted in the ChubTur^{®} cells (sterile Ringer's solution).
iii) ChubTur^{®} cells were incubated at 20 - 25°C for 14 days. Negative controls were set up where the nails were not infected with any test organism or treated with any formulation.
iv) After 14 days, the ChubTur^{®} cells were removed from incubation at 20 - 25°C and the formulation was individually applied to the surface of the nail, opposite to where the nail was inoculated with the organism suspension (Fig. 1b).
v) The ChubTur^{®} cells were removed from 20 - 25°C incubation and the nails were dismantled from the gasket of the ChubTur^{®} cells. The nails were completely cleaned of all residual formulation from the nail surface by employing the following steps:
   a. The surface of the nail was wiped with a dry sterile cotton swab
   b. Following step a., the surface of the nail was wiped with a cotton swab dampened in Ringer's solution. This was repeated with a second swab dampened in Ringer's solution
   c. The surface of the nail was cleaned with a final dry swab to remove any moisture from the surface of the nail
   d. The nail was left to air dry for a further 30 min under a biological safety cabinet
vi) The nails were analyzed for the presence of ATP from the viable fungi using the method described before

In this experiment the nails were treated once daily with 2 µl test solution over 7 days, respectively.

3 abafungin concentrations were tested in a Liqui-Patch^{®} formulation (Table 5):

**Table 5:**

| | | | |
|---|---|---|---|
| abafungin | 2 % by weight | 5 % by weight | 10 % by weight |
| glacial acetic acid | 6 % by weight | 6 % by weight | 6 % by weight |
| ethyl acetate | 10 % by weight | 10 % by weight | 20 % by weight |
| Liqui-Patch^{®} | 82 % by weight | 79 % by weight | 64 % by weight |

A 10% eficonazole formulation (Jublia^{™}) was used as positive control (see above). The detected amount for the infected untreated nails was set to 100% and the amounts of detected ATP were normalized to that.

Table 6 indicates the test solutions and the percentage of determined ATP, in comparison to infected untreated nails (mean ± SEM).

**Table 6:**

| | | |
|---|---|---|
| 1. | 2% abafungin | 7.7 ± 2.4 % |
| 2. | 5% abafungin | 3.2 ± 1.4 % |
| 3. | 10% abafungin | 1.1 ± 0.3 % |
| 4. | 10% eficonazole (Jublia^{™}) | 5.6 ± 3.0 % |
| 5. | infected untreated control | 100 ± 36.6 % |
| 6. | not infected | 0.8 ± 0.2 % |

As shown in Table 6, abafungin in the Liqui-Patch^{®} formulation according to the disclosure is highly effective to suppress *T. rubrum* in this model. Moreover, there is a dose-dependency. This is a strong argument against an unspecific cytotoxic effect. In comparison with eficonazole, abafungin in the Liqui-Patch^{®} formulation according to the disclosure seems to be even more effective than the comparator Jublia^{™}.

### FIGURES

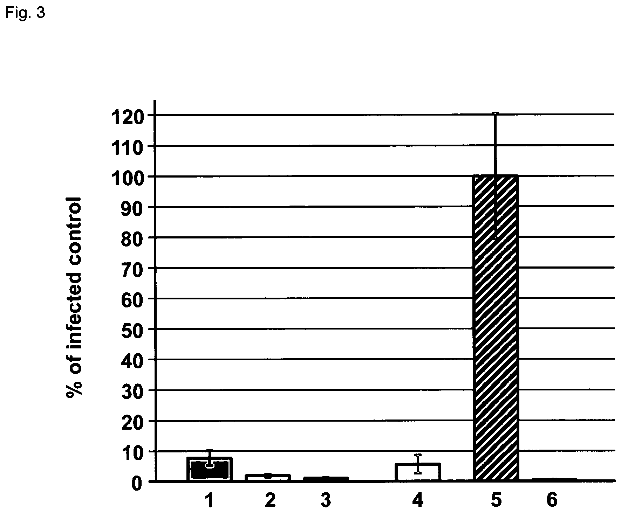

- Fig. 1A:: Schematic representation of a human distal nail clipping, with broken lines indicating examples of areas where the nail was cut in order to obtain ca. 3 mm x 3 mm (ca. 9 mm² surface area) samples
- Fig. 1B:: Schematic representation of human nail mounted in a gasket with the relative position of the formulation and organism indicated
1: formulation
2: nail
3: fungi
4: nail gasket
- Fig. 2:: Evaluation of Example 5. The zone-of-inhibition of the infected untreated nails was taken as 100%. Mean ± SEM (ThurChub^{®})
1: 5% abafungin in a liquid-patch formulation
2: 10% eficonazole (Jublia^{™})
3: infected, untreated control
4: not infected
- Fig. 3:: Evaluation of Example 6. The ATP content of the infected untreated nails was taken as 100%. Mean ± SEM. (RoMar^{®})
1: 2% abafungin in a liquid-patch formulation
2: 5% abafungin in a liquid-patch formulation
3: 10% abafungin in a liquid-patch formulation
4: 10% eficonazole (Jublia^{™})
5: infected, untreated control
6: not infected

## Claims

1. Pharmaceutical composition, comprising
a) at least one substituted 2-aminothiazole or of its pharmaceutically acceptable salts of the general formula (I) 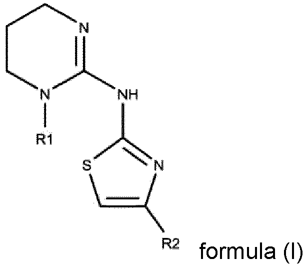
wherein R₁ represents hydrogen or C₁-C₄ straight chain or branched alkyl and R₂ represents a radical a), b, or c) of the general formulas (II) 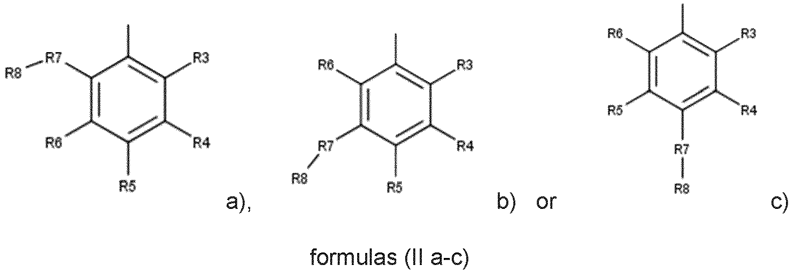
wherein R₃, R₄, R₅ and R₆ independently of one another in each case represent hydrogen, halogen, nitro, alkyl, alkoxy, alkoxycarbonyl, dialkylamino, alkylthio, alkylsulfinyl, alkylsulfonyl each having 1 to 4 carbon atoms in the respective alkyl moieties, or halogenoalkyl, halogenoalkoxy, halogenoalkylthio, halogenoalkylsulfinyl or halogenoalkylsulfonyl each having 1 to 4 carbon atoms and 1 to 9 halogen atoms,
R₇ represents oxygen, sulfur, sulfinyl or sulfonyl and R₈ represents an unsubstituted C₆-C₁₀ aryl or a substituted C₆-C₁₀ aryl radical, substituted by halogen or alkyl, alkoxy, alkoxycarbonyl, dialkylamino, alkylthio, alkylsulphinyl or alkylsulphonyl each having 1 to 8 carbon atoms in the respective alkyl moieties, or halogenoalkyl, halogenoalkoxy, halogenoalkylthio, halogenoalkylsulphinyl or halogenoalkylsulphonyl each having 1 to 4 carbon atoms and 1 to 9 halogen atoms, cycloalkyl having 3 to 7 carbon atoms, phenylalkyl or phenoxyalkyl each having 1 to 4 carbon atoms in the alkyl moiety and also phenyl or phenoxy, a tautomer thereof, or a pharmaceutically acceptable acid addition salt thereof;
b) a completely reacted, carboxylated linear polyurethane, comprising the reaction product that consists of
(1) one or more 2,2-hydroxymethyl-substituted carboxylic acid(s), produced by the general formula (IV), 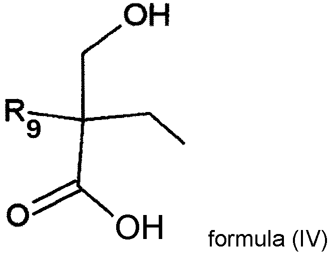 in which R₉ means hydrogen or C₁-C₂₀ alkyl, present in an amount by weight that is sufficient to produce 0.35-2.25 milliequivalents of carboxyl functionality per gram of polyurethane,
(2) 10-90% by weight, relative to the weight of polyurethane, of one or more organic compound(s), which in each case do not have more than two active hydrogen atoms, and
(3) one or more organic diisocyanate(s), present in an amount that is sufficient to react with the active hydrogen atoms of the 2,2-hydroxymethyl-substituted carboxylic acid and the organic compounds, with the exception of hydrogen, to form the carboxylate of the 2,2-hydroxymethyl-substituted carboxylic acid;
c) at least one organic or inorganic acid, optionally a methyl, ethyl, propyl or isopropyl ester of said acid(s);
d) an organic solvent;
e) a C₂-C₄ alcohol;
f) H₂O;
g) a plasticizer; and
h) optionally, at least one pharmaceutically acceptable excipient, and/or at least one propellent.

2. Pharmaceutical composition according to claim 1, wherein the components a) to h) have the following ranges in the total composition:
a) at least one substituted 2-aminothiazole or of its pharmaceutically acceptable salts in the overall range of 0.5 % to 15% per weight,
b) completely reacted, carboxylated linear polyurethane in the overall range of 0.5% to 4% per weight,
c) at least one organic or inorganic acid in the overall range of 3% to 8% per weight,
d) organic solvent in the overall range of 5% to 25% per weight,
e) C₂-C₄ alcohol in the overall range of 40% to 75% per weight,
f) H₂O in the overall range of 7% to 14% per weight,
g) plasticizer in the overall range of 0.5% to 0.85% per weight, and
h) at least one pharmaceutically acceptable excipient, and/or at least one propellent in the overall range of 0% to 15% per weight,
wherein the relative weight percentages of all ingredients add up to 100 %.

3. Pharmaceutical composition according to any one of claim 1 or 2, wherein the at least one substituted 2-aminothiazole is 4-[2-(2,4-dimethylphenoxy)phenyl]-N-(1,4,5,6-tetrahydropyrimidin-2-yl)-1,3-thiazol-2-amine.

4. Pharmaceutical composition according to any one of claim 1 to 3, wherein the completely reacted, carboxylated linear polyurethane is polyurethane-14-AMP-acrylate copolymer.

5. Pharmaceutical composition according to any one of claims 1 to 4, wherein the least one of organic or inorganic acid is selected from the group consisting of hydrochloric acid, trichloroacetic acid, boric acid, phosphoric acid, formic acid, acetic acid, glyoxylic acid, pyruvic acid, fumaric acid, ascorbic acid, oxalic acid, tannic acid, propionic acid, glycolic acid, lactic acid, lauric acid, or mixtures thereof.

6. Pharmaceutical composition according to any one of claims 1 to 5, wherein the organic solvent is selected from the group consisting of N-methyl pyrrolidone, DMSO, ethyl acetate, butyl acetate, PEG 600, or mixtures thereof.

7. Pharmaceutical composition according to any one of claims 1 to 6, wherein the C₂-C₄ alcohol is selected from the group consisting of ethanol, glycol, 1-propanol, 2-propanol, propane-1,2-diol, propane-1,3-diol, glycerol, 1-butanol, 2-butanol, 1,2-butanediol, 1,3-butanediol,1,4-butanediol, or mixtures thereof.

8. Pharmaceutical composition according to any one of claims 1 to 7, wherein the plasticizer is selected from the group consisting of triethyl citrate, tributyl citrate, acetyl triethyl citrate, acetyl tributyl citrate, triacetin, dibutyl phthalate, tributyl sebacate, diethyl phthalate, propylene glycol, polyethylene glycol, glycerol, castor oil, or mixtures thereof.

9. Pharmaceutical composition according to any one of claims 1 to 8, wherein the composition additionally contains a propellant selected from a group consisting of dimethyl ether, propane, butane, air, nitrogen, or mixtures thereof.

10. Pharmaceutical composition according to any one of claims 1 to 9, wherein the composition additionally contains an excipient selected from a group consisting of moisturizers, emulsifiers, stabilizers, solubilizers, permeation enhancers, colorants, preservatives, antioxidants and aromatic substances.

11. Pharmaceutical composition according to any one of claims 1 to 10 for use in the prophylaxis or treatment of onychomycosis.

12. Pharmaceutical composition for use according to claim 11, wherein the pharmaceutical composition is suitable for topical use.

13. Pharmaceutical composition for use according to any one of claim 11 or 12, wherein the pharmaceutical composition is suitable for application, spraying or brushing onto a nail suffering from onychomycosis.

14. Method for preparing a pharmaceutical composition as disclosed in any one of claims 1 to 8 or 10, comprising the following steps:
a) at least one substituted 2-aminothiazole as defined in claim 1 and at least one organic or inorganic acid as defined in claim 5 are mixed under stirring to afford a first solution;
b) the first solution of step a) is mixed under stirring with an organic solvent as defined in claim 6 to afford a second solution;
c) a completely reacted, carboxylated linear polyurethane as defined in claim 1, a C₂-C₄ alcohol as defined in claim 7, H₂O and a plasticizer as defined in claim 8 are mixed under stirring to afford a third solution;
d) the second solution of step b) and the third solution of step c) are mixed under stirring to afford a fourth solution; and
e) optionally, at least one pharmaceutically acceptable excipient as defined in claim 9 or 10 is added to the fourth solution of step d).

## Patentansprüche

1. Pharmazeutische Zusammensetzung, umfassend
a) mindestens ein substituiertes 2-Aminothiazol oder eines seiner pharmazeutisch verträglichen Salze der allgemeinen Formel (I) 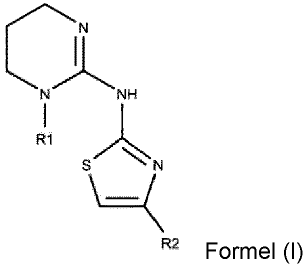
wobei R₁ für Wasserstoff oder ein C₁-C₄ geradkettiges oder verzweigtes Alkyl und R₂ für ein Radikal a), b), oder c) der allgemeinen Formeln (II) steht, 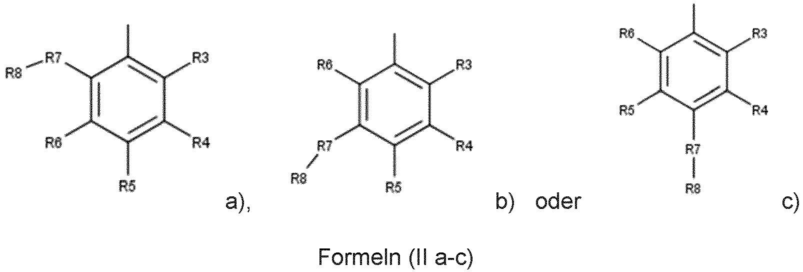
wobei R₃, R₄, R₅ und R₆ unabhängig voneinander jeweils für Wasserstoff, Halogen, Nitro, Alkyl, Alkoxy, Alkoxycarbonyl, Dialkylamino, Alkylthio, Alkylsulfinyl, Alkylsulfonyl mit jeweils 1 bis 4 Kohlenstoffatomen in den jeweiligen Alkyresten, oder Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Halogenatomen stehen,
R₇ für Sauerstoff, Schwefel, Sulfinyl oder Sulfonyl steht und R₈ für ein nicht substituiertes C₆-C₁₀ Aryl oder ein substituiertes C₆-C₁₀ Arylradikal steht, das durch Halogen oder Alkyl, Alkoxy, Alkoxycarbonyl, Dialkylamino, Alkylthio, Alkylsulphinyl oder Alkylsulphonyl mit jeweils 1 bis 8 Kohlenstoffatomen in den jeweiligen Alkylresten subsituiert ist, oder für Halogenalkyl, Halogenalkoxy, Halogenalkylthio, Halogenalkylsulphinyl oder Halogenalkylsulphonyl mit jeweils 1 bis 4 Kohlenstoffatomen und 1 bis 9 Halogenatomen, Cycloalkyl mit jeweils 3 bis 7 Kohlenstoffatomen, Phenylalkyl oder Phenoxyalkyl mit jeweils 1 bis 4 Kohlenstoffatomen in dem Alkylrest und auch für Phenyl oder Phenoxy, ein Tautomer davon, oder für ein pharmazeutisch verträgliches Additionssalz davon steht;
b) ein vollständig umgesetztes, carboxyliertes, lineares Polyurethan, das das Reaktionsprodukt umfasst, das besteht aus
(1) einer oder mehreren 2,2-Hydroxymethyl-substituierten Carbonsäure(n), nach der allgemeinen Formel (IV) hergestellt, 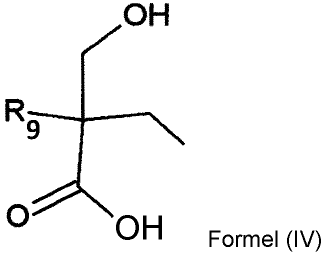 wobei R₉ für Wasserstoff oder C₁-C₂₀ Alkyl steht, das in einem Gewichtsverhältnis vorliegt, das ausreicht, um 0,35-2,25 Milliäquivalente an Carboxylfunktionalität pro Gramm Polyurethan herzustellen,
(2) 10-90 Gew.-%, relativ zu dem Polyurethangewicht, einer oder mehrerer organischer Verbindung(en), die jeweils nicht mehr als zwei aktive Wasserstoffatome haben, und
(3) ein oder mehrere organische Diisocyanat(e), die in einer Menge vorliegen, die ausreichend ist, um mit den aktiven Wasserstoffatomen der 2,2-Hydroxymethyl-substituierten Carbonsäure und der organischen Verbindungen zu reagieren, mit der Ausnahme von Wasserstoff, um das Carboxylat der 2,2-Hydroxymethyl-substituierten Carbonsäure zu bilden;
c) mindestens eine organische oder anorganische Säure, optional ein Methyl-, Ethyl-, Propyl- oder Isopropylester der besagten Säure(n);
d) ein organisches Lösungsmittel;
e) ein C₂-C₄ Alkohol;
f) H₂O;
g) ein Weichmacher; und
h) optional, mindestens ein pharmazeutisch verträglicher Hilfsstoff, und/oder mindestens ein Treibmittel.

2. Pharmazeutische Zusammensetzung gemäß Anspruch 1, wobei die Komponenten a) bis h) die folgenden Bereiche an der Gesamtzusammensetzung aufweisen:
a) mindestens ein substituiertes 2-Aminothiazol oder eines seiner pharmazeutisch verträglichen Salze im Gesamtbereich von 0,5 Gew.-% bis 15 Gew.-%,
b) vollständig umgesetztes, carboxyliertes, lineares Polyurethan im Gesamtbereich von 0,5 Gew.-% bis 4 Gew.-%,
c) mindestens eine organische oder anorganische Säure im Gesamtbereich von 3 Gew.-% bis 8 Gew.-%,
d) organisches Lösungsmittel im Gesamtbereich von 5 Gew.-% bis 25 Gew.-%,
e) C₂-C₄ Alkohol im Gesamtbereich von 40 Gew.-% bis 75 Gew.-%,
f) H₂O im Gesamtbereich von 7 Gew.-% bis 14 Gew.-%,
g) Weichmacher im Gesamtbereich von 0,5 Gew.-% bis 0,85 Gew.-%, und
h) mindestens ein pharmazeutisch verträglicher Hilfsstoff und/oder mindestens ein Treibmittel im Gesamtbereich von 0 Gew.-% bis 15 Gew.-%,
wobei sich die relativen Gewichtsanteile aller Bestandteile zu 100 % addieren.

3. Pharmazeutische Zusammensetzung gemäß einem der Ansprüche 1 oder 2, wobei das mindestens eine substituierte 2-Aminothiazol 4-[2-(2,4-Dimethylphenoxy)phenyl]-N-(1,4,5,6-tetrahydropyrimidin-2-yl)-1,3-thiazol-2-amin ist.

4. Pharmazeutische Zusammensetzung gemäß einem der Ansprüche 1 bis 3, wobei das vollständig umgesetzte, carboxylierte, lineare Polyurethan Polyurethan-14-AMP-Acrylat Co-Polymer ist.

5. Pharmazeutische Zusammensetzung gemäß einem der Ansprüche 1 bis 4, wobei die mindestens eine organische oder anorganische Säure aus der Gruppe ausgewählt wird, die aus Salzsäure, Trichloressigsäure, Borsäure, Phosphorsäure, Ameisensäure, Essigsäure, Glyoxalsäure, Brenztraubensäure, Fumarsäure, Ascorbinsäure, Oxalsäure, Gerbsäure, Propionsäure, Glykolsäure, Milchsäure, Laurinsäure oder deren Gemischen besteht.

6. Pharmazeutische Zusammensetzung gemäß einem der Ansprüche 1 bis 5, wobei das organische Lösungsmittel aus der Gruppe ausgewählt wird, die aus N-Methylpyrrolidon, DMSO, Ethylacetat, Butylacetat, PEG 600 oder deren Gemischen besteht.

7. Pharmazeutische Zusammensetzung gemäß einem der Ansprüche 1 bis 6, wobei der C₂-C₄ Alkohol aus der Gruppe ausgewählt wird, die aus Ethanol, Glykol, 1-Propanol, 2-Propanol, Propan-1,2-diol, Propan-1,3-diol, Glycerin, 1-Butanol, 2-Butanol, 1,2-Butandiol, 1,3-Butandiol, 1,4-Butandiol oder deren Gemischen besteht.

8. Pharmazeutische Zusammensetzung gemäß einem der Ansprüche 1 bis 7, wobei der Weichmacher aus der Gruppe ausgewählt wird, die aus Triethylcitrat, Tributylcitrat, Acetyltriethylcitrat, Acetyltributylcitrat, Triacetin, Dibutylphthalat, Tributylsebacat, Diethylphthalat, Propylenglykol, Polyethylenglykol, Glycerin, Rizinusöl oder deren Gemischen besteht.

9. Pharmazeutische Zusammensetzung gemäß einem der Ansprüche 1 bis 8, wobei die Zusammensetzung zusätzlich ein Treibmittel enthält, das aus der Gruppe ausgewählt wird, die aus Dimethylether, Propan, Butan, Luft, Stickstoff oder deren Gemischen besteht.

10. Pharmazeutische Zusammensetzung gemäß einem der Ansprüche 1 bis 9, wobei die Zusammensetzung zusätzlich einen Hilfsstoff enthält, der aus der Gruppe ausgewählt wird, die aus Feuchthaltemitteln, Emulgatoren, Stabilisierungsmitteln, Lösungsvermittlern, Permeationsverstärkern, Farbstoffen, Konservierungsmitteln, Antioxidantien und Aromastoffen besteht.

11. Pharmazeutische Zusammensetzung gemäß einem der Ansprüche 1 bis 10 zur Verwendung in der Prophylaxe oder Behandlung von Onychomykosen.

12. Pharmazeutische Zusammensetzung gemäß Anspruch 11, wobei die pharmazeutische Zusammensetzung für eine topische Anwendung geeignet ist.

13. Pharmazeutische Zusammensetzung gemäß einem der Ansprüche 11 oder 12, wobei die pharmazeutische Zusammensetzung für eine Applikation, Besprühen oder Bepinseln auf einen Nagel geeignet ist, der an Onychomykose leidet.

14. Verfahren zur Herstellung einer pharmazeutischen Zusammensetzung wie in einem der Ansprüche 1 bis 8 oder 10 dargelegt, das die folgenden Schritte umfasst:
a) mindestens ein substituiertes 2-Aminothiazol wie in Anspruch 1 definiert und mindestens eine organische oder anorganische Säure wie in Anspruch 5 definiert werden unter Rühren gemischt, um eine erste Lösung zu erzeugen;
b) die erste Lösung aus Schritt a) wird unter Rühren mit einem organischen Lösungsmittel wie in Anspruch 6 definiert gemischt, um eine zweite Lösung zu erzeugen;
c) ein vollständig umgesetztes, carboxyliertes, lineares Polyurethan wie in Anspruch 1 definiert, ein C₂-C₄ Alkohol wie in Anspruch 7 definiert, H₂O und ein Weichmacher wie in Anspruch 8 definiert werden unter Rühren gemischt, um eine dritte Lösung zu erzeugen;
d) die zweite Lösung aus Schritt b) und die dritte Lösung aus Schritt c) werden unter Rühren gemischt, um eine vierte Lösung zu erzeugen; und
e) optional wird mindestens ein pharmazeutisch verträglicher Hilfsstoff wie in Anspruch 9 oder 10 definiert zu der vierten Lösung aus Schritt d) hinzugegeben.

## Revendications

1. Composition pharmaceutique, comprenant
a) au moins un 2-aminothiazole substitué ou l'un de ses sels pharmaceutiquement acceptables de formule générale (I) 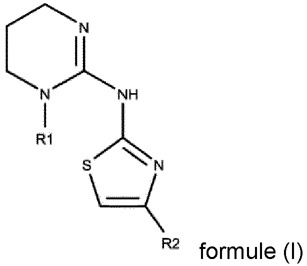
dans laquelle R₁ représente l'hydrogène ou un alkyle à chaîne linéaire ou ramifiée en C₁-C₄ et R₂ représente un radical a), b) ou c) des formules générales (II) 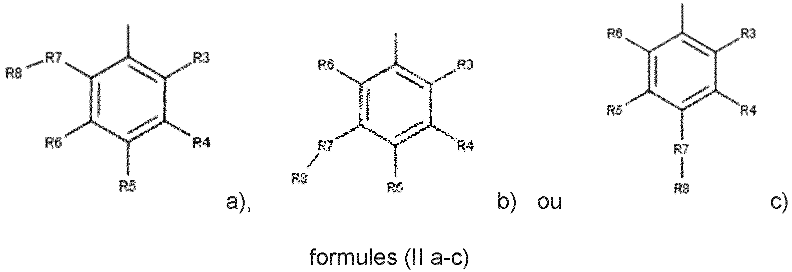
dans lesquelles R₃, R₄, R₅ et R₆ représentent indépendamment l'un de l'autre l'hydrogène, l'halogène, le nitro, l'alkyle, l'alcoxy, l'alcoxycarbonyle, le dialkylamino, l'alkylthio, l'alkylsulfinyl, l'alkylsulfonyle ayant chacun 1 à 4 atomes de carbone dans les groupes alkyles respectives, ou l'halogénoalkyle, l'halogénoalcoxy, l'halogénoalkylthio, l'halogénoalkylsulfinyle ou l'halogénoalkylsulfonyle ayant chacun 1 à 4 atomes de carbone et 1 à 9 atomes d'halogène,
R₇ représente l'oxygène, le soufre, le sulfinyle ou le sulfonyle et R₈ représente un aryle en C₆-C₁₀ non substitué ou un radical aryle en C₆-C₁₀ substitué par un halogène ou un alkyle, un alcoxy, un alcoxycarbonyle, un dialkylamino, un alkylthio, un alkylsulphinyle ou un alkylsulphonyle ayant chacun de 1 à 8 atomes de carbone dans les groupes alkyles respectives, ou un halogénoalkyle, un halogénoalcoxy, un halogénoalkylthio, un halogénoalkylsulphinyle ou un halogénoalkylsulphonyle ayant chacun 1 à 4 atomes de carbone et 1 à 9 atomes d'halogène, un cycloalkyle ayant 3 à 7 atomes de carbone, un phénylalkyle ou un phénoxyalkyle ayant chacun 1 à 4 atomes de carbone dans le groupe alkyle et également un phényle ou un phénoxy, un tautomère de ceux-ci, ou un sel d'addition d'acide pharmaceutiquement acceptable de ceux-ci;
b) un polyuréthane linéaire carboxylé ayant complètement réagi, comprenant le produit de la réaction qui consiste en
(1) un ou plusieurs acides carboxyliques substitués par 2,2-hydroxyméthyle, obtenus par la formule générale (IV), 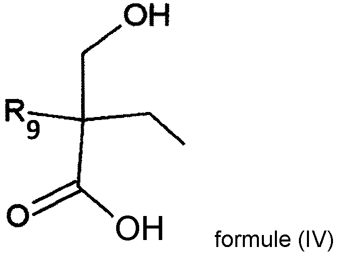 dans laquelle R₉ signifie l'hydrogène ou l'alkyle en C₁-C₂₀, présent en une quantité en poids suffisante pour produire 0,35-2,25 milliéquivalents de fonctionnalité carboxyle par gramme de polyuréthane,
(2) 10-90% en poids, par rapport au poids du polyuréthane, d'un ou de plusieurs composés organiques qui, dans chaque cas, n'ont pas plus de deux atomes d'hydrogène actifs, et
(3) un ou plusieurs diisocyanate(s) organique(s), présents en quantité suffisante pour réagir avec les atomes d'hydrogène actifs de l'acide carboxylique 2,2-hydroxyméthyle-substitué et les composés organiques, à l'exception de l'hydrogène, pour former le carboxylate de l'acide carboxylique 2,2-hydroxyméthyle-substitué;
c) au moins un acide organique ou inorganique, optionnellement un ester de méthyle, d'éthyle, de propyle ou d'isopropyle dudit (desdits) acide(s);
d) un solvant organique;
e) un alcool en C₂-C₄;
f) H₂O;
g) un plastifiant; et
h) optionnellement, au moins un excipient pharmaceutiquement acceptable, et/ou au moins un agent propulseur.

2. Composition pharmaceutique selon la revendication 1, dans laquelle les composants a) à h) se répartissent comme suit dans la composition totale:
a) au moins un 2-aminothiazole substitué ou l'un de ses sels pharmaceutiquement acceptables dans une gamme globale de 0,5 % à 15 % en poids,
b) un polyuréthane linéaire carboxylé ayant complètement réagi, dans une gamme globale de 0,5 % à 4 % en poids,
c) au moins un acide organique ou inorganique dans une gamme globale de 3 % à 8 % en poids,
d) un solvant organique dans une gamme globale de 5 % à 25 % en poids,
e) un alcool en C₂-C₄ dans une gamme globale de 40 % à 75 % en poids,
f) H₂O dans une gamme globale de 7 % à 14 % en poids,
g) un plastifiant dans une gamme globale de 0,5 % à 0,85% en poids, et
h) au moins un excipient pharmaceutiquement acceptable et/ou au moins un agent propulseur dans une gamme globale de 0 % à 15 % en poids,
dans laquelle les pourcentages de poids relatifs de tous les ingrédients s'élèvent à 100 %.

3. Composition pharmaceutique selon l'une quelconque des revendications 1 ou 2, dans laquelle l'au moins un 2-aminothiazole substitué est le 4-[2-(2,4-diméthylphénoxy)phényl]-N-(1,4,5,6-tétrahydropyrimidin-2-yl)-1,3-thiazol-2-amine.

4. Composition pharmaceutique selon l'une quelconque des revendications 1 à 3, dans laquelle le polyuréthane linéaire carboxylé ayant complètement réagi est un copolymère polyuréthane-14-AMP-acrylate.

5. Composition pharmaceutique selon l'une quelconque des revendications 1 à 4, dans laquelle au moins un acide organique ou inorganique est choisi dans le groupe qui consiste en l'acide chlorhydrique, l'acide trichloracétique, l'acide borique, l'acide phosphorique, l'acide formique, l'acide acétique, l'acide glyoxylique, l'acide pyruvique, l'acide fumarique, l'acide ascorbique, l'acide oxalique, l'acide tannique, l'acide propionique, l'acide glycolique, l'acide lactique, l'acide laurique, ou des mélanges de ces acides.

6. Composition pharmaceutique selon l'une quelconque des revendications 1 à 5, dans laquelle le solvant organique est choisi dans le groupe qui consiste en la N-méthyl pyrrolidone, le DMSO, l'acétate d'éthyle, l'acétate de butyle, le PEG 600, ou leurs mélanges.

7. Composition pharmaceutique selon l'une quelconque des revendications 1 à 6, dans laquelle l'alcool en C₂-C₄ est choisi dans le groupe qui consiste en l'éthanol, le glycol, le 1-propanol, le 2-propanol, le propane-1,2-diol, le propane-1,3-diol, le glycérol, le 1-butanol, le 2-butanol, le 1,2-butanediol, le 1,3-butanediol, le 1,4-butanediol, ou leurs mélanges.

8. Composition pharmaceutique selon l'une quelconque des revendications 1 à 7, dans laquelle le plastifiant est choisi dans le groupe qui consiste en le citrate de triéthyle, le citrate de tributyle, le citrate d'acétyle triéthyle, le citrate d'acétyle tributyle, la triacétine, le phtalate de dibutyle, le sébacate de tributyle, le phtalate de diéthyle, le propylène glycol, le polyéthylène glycol, le glycérol, l'huile de ricin, ou leurs mélanges.

9. Composition pharmaceutique selon l'une quelconque des revendications 1 à 8, dans laquelle la composition contient en outre un agent propulseur choisi dans un groupe qui consiste en l'éther diméthylique, le propane, le butane, l'air, l'azote, ou leurs mélanges.

10. Composition pharmaceutique selon l'une quelconque des revendications 1 à 9, dans laquelle la composition contient en outre un excipient choisi dans un groupe qui consiste en des hydratants, des émulsifiants, des stabilisants, des solubilisants, des améliorateurs de perméation, des colorants, des conservateurs, des antioxydants et des substances aromatiques.

11. Composition pharmaceutique selon l'une quelconque des revendications 1 à 10 pour utilisation dans la prophylaxie ou le traitement de l'onychomycose.

12. Composition pharmaceutique pour utilisation selon la revendication 11, dans laquelle la composition pharmaceutique convient à un usage topique.

13. Composition pharmaceutique pour utilisation selon l'une quelconque des revendications 11 ou 12, dans laquelle la composition pharmaceutique convient à l'application, à la pulvérisation ou au brossage sur un ongle souffrant d'onychomycose.

14. Procédé de préparation d'une composition pharmaceutique telle que décrite dans l'une quelconque des revendications 1 à 8 ou 10, comprenant les étapes suivantes :
a) au moins un 2-aminothiazole substitué tel que défini dans la revendication 1 et au moins un acide organique ou inorganique tel que défini dans la revendication 5 sont mélangés sous agitation pour obtenir une première solution;
b) la première solution de l'étape a) est mélangée sous agitation avec un solvant organique tel que défini dans la revendication 6 pour obtenir une deuxième solution;
c) un polyuréthane linéaire carboxylé ayant complètement réagi tel que défini dans la revendication 1, un alcool en C₂-C₄ tel que défini dans la revendication 7, H₂O et un plastifiant tel que défini dans la revendication 8 sont mélangés sous agitation pour obtenir une troisième solution;
d) la deuxième solution de l'étape b) et la troisième solution de l'étape c) sont mélangées sous agitation pour obtenir une quatrième solution; et
e) optionnellement, au moins un excipient pharmaceutiquement acceptable tel que défini dans la revendication 9 ou 10 est ajouté à la quatrième solution de l'étape d).
